# EUROPEAN PATENT SPECIFICATION

(11) **EP 2 512 225 B1**
(45) Date of publication and mention of the grant of the patent: **23.08.2017**
(21) Application number: 10842622.2
(22) Date of filing: 16.12.2010
(51) Int. Cl.: A01H 5/00, C07K 14/325, C12N 15/82

(54) **USE OF VIP3AB IN COMBINATION WITH CRY1CA FOR MANAGEMENT OF RESISTANT INSECTS**
VERWENDUNG VON VIP3AB IN KOMBINATION MIT CRY1CA ZUR BEKÄMPFUNG RESISTENTER INSEKTEN
UTILISATION DE VIP3AB COMBINÉE À CRY1CA POUR LA GESTION DES INSECTES RÉSISTANTS

(30) Priority: 16.12.2009 US 284275 P; 16.12.2009 US 284281 P
(43) Date of publication of application: 24.10.2012
(73) Proprietor: Dow AgroSciences, LLC, Indianapolis, IN 46268 (US)
(72) Inventor: MEADE, Thomas, Zionsville IN 46077 (US); NARVA, Kenneth, Zionsville IN 46077 (US); STORER, Nicholas, P., Kensington MD 20895 (US); SHEETS, Joel, J., Zionsville IN 46077 (US); WOOSLEY, Aaron, T., Fishers IN 46038 (US); BURTON, Stephanie, L., Indianapolis IN 46278 (US)
(74) Representative: Zwicker, Jörk
(86) International application number: PCT/US2010/060835
(87) International publication number: WO 2011/084634

(56) References cited:
- WO-A1-2009/132850
- US-A1- 2004 128 716
- US-A1- 2004 197 916
- US-A1- 2009 038 030
- US-A1- 2009 222 947
- , 1 January 2009 (2009-01-01), XP55060016, Retrieved from the Internet: URL:http://www.agry.purdue.edu/CCA/2008/Pr oceedings/Steffey.pdf [retrieved on 2013-04-17]
- PITTENDRIGH B R ET AL: "''Active'' refuges can inhibit the evolution of resistance in insects towards transgenic insect-resistant plants", JOURNAL OF THEORETICAL BIOLOGY, ACADEMIC PRESS, LONDON, GB, vol. 231, no. 4, 21 December 2004 (2004-12-21), pages 461-474, XP004635702, ISSN: 0022-5193, DOI: 10.1016/J.JTBI.2004.05.023
- , 1 December 2009 (2009-12-01), XP55060013, Retrieved from the Internet: URL:http://combat-monsanto.org/IMG/pdf/btc rops.pdf [retrieved on 2013-04-17]
- , 9 December 2008 (2008-12-09), XP55060010, DOI: 10.1094/PHP-2004- Retrieved from the Internet: URL:http://www.pnas.org/content/105/49/190 29.full.pdf#page=1&view=FitH [retrieved on 2013-04-17]
- MOAR W J: "Breathing new life into insect-resistant plants", NATURE BIOTECHNOLOGY, NATURE PUBLISHING GROUP, US, vol. 21, no. 10, 1 October 2003 (2003-10-01), pages 1152-1154, XP002319567, ISSN: 1087-0156, DOI: 10.1038/NBT1003-1152
- LEE M K ET AL: "The mode of action of the Bacillus thuringiensis vegetative insecticidal protein Vip3A differs from that of Cry1Ab [delta]-endotoxin", APPLIED AND ENVIRONMENTAL MICROBIOLOGY, AMERICAN SOCIETY FOR MICROBIOLOGY, US, vol. 69, no. 8, 1 August 2003 (2003-08-01) , pages 4648-4657, XP002536190, ISSN: 0099-2240, DOI: 10.1128/AEM.69.8.4648-4657.2003

### SEQUENCE LISTING

<110> Dow AGROSCIENCES LLC
<120> COMBINED USE OF VIP3AB1 AND CRY1CA FOR MANAGEMENT OF RESISTANT INSECTS
<130> DAS-P0162-US-07
<160> 2
<170> PatentIn version 3.5
<210> 1
   <211> 788
   <212> PRT
   <213> Artificial Sequence
<220>
   <223> Vip3Ab1
<400> 1
   Met Ala Asn Met Asn Asn Thr Lys Leu Asn Ala Arg Ala Leu Pro Ser
   1               5                   10                  15
   Phe Ile Asp Tyr Phe Asn Gly Ile Tyr Gly Phe Ala Thr Gly Ile Lys
               20                  25                  30
   Asp Ile Met Asn Met Ile Phe Lys Thr Asp Thr Gly Gly Asn Leu Thr
           35                  40                  45
   Leu Asp Glu Ile Leu Lys Asn Gln Gln Leu Leu Asn Glu Ile Ser Gly
       50                  55                  60
   Lys Leu Asp Gly Val Asn Gly Ser Leu Asn Asp Leu Ile Ala Gln Gly
   65                  70                  75                  80
   Asn Leu Asn Thr Glu Leu Ser Lys Glu Ile Leu Lys Ile Ala Asn Glu
                   85                  90                  95
   Gln Asn Gln Val Leu Asn Asp Val Asn Asn Lys Leu Asp Ala Ile Asn
               100                 105                 110
   Thr Met Leu His Ile Tyr Leu Pro Lys Ile Thr Ser Met Leu Ser Asp
           115                 120                 125
   Val Met Lys Gln Asn Tyr Ala Leu Ser Leu Gln Val Glu Tyr Leu Ser
       130                 135                 140
   Lys Gln Leu Lys Glu Ile Ser Asp Lys Leu Asp Val Ile Asn Val Asn
   145                 150                 155                 160
   Val Leu Ile Asn Ser Thr Leu Thr Glu Ile Thr Pro Ala Tyr Gln Arg
                   165                 170                 175
   Ile Lys Tyr Val Asn Glu Lys Phe Glu Glu Leu Thr Phe Ala Thr Glu
               180                 185                 190
   Thr Thr Leu Lys Val Lys Lys Asp Ser Ser Pro Ala Asp Ile Leu Asp
           195                 200                 205
   Glu Leu Thr Glu Leu Thr Glu Leu Ala Lys Ser Val Thr Lys Asn Asp
       210                 215                 220
   Val Asp Gly Phe Glu Phe Tyr Leu Asn Thr Phe His Asp Val Met Val
   225                 230                 235                 240
   Gly Asn Asn Leu Phe Gly Arg Ser Ala Leu Lys Thr Ala Ser Glu Leu
                   245                 250                 255
   Ile Ala Lys Glu Asn Val Lys Thr Ser Gly Ser Glu Val Gly Asn Val
               260                 265                 270
   Tyr Asn Phe Leu Ile Val Leu Thr Ala Leu Gln Ala Lys Ala Phe Leu
           275                 280                 285
   Thr Leu Thr Thr Cys Arg Lys Leu Leu Gly Leu Ala Asp Ile Asp Tyr
       290                 295                 300
   Thr Ser Ile Met Asn Glu His Leu Asn Lys Glu Lys Glu Glu Phe Arg
   305                 310                 315                 320
   Val Asn Ile Leu Pro Thr Leu Ser Asn Thr Phe Ser Asn Pro Asn Tyr
                   325                 330                 335
   Ala Lys Val Lys Gly Ser Asp Glu Asp Ala Lys Met Ile Val Glu Ala
               340                 345                 350
   Lys Pro Gly His Ala Leu Val Gly Phe Glu Ile Ser Asn Asp Ser Met
           355                 360                 365
   Thr Val Leu Lys Val Tyr Glu Ala Lys Leu Lys Gln Asn Tyr Gln Val
       370                 375                 380
   Asp Lys Asp Ser Leu Ser Glu Val Ile Tyr Ser Asp Met Asp Lys Leu
   385                 390                 395                 400
   Leu Cys Pro Asp Gln Ser Glu Gln Ile Tyr Tyr Thr Asn Asn Ile Val
                   405                 410                 415
   Phe Pro Asn Glu Tyr Val Ile Thr Lys Ile Asp Phe Thr Lys Lys Met
               420                 425                 430
   Lys Thr Leu Arg Tyr Glu Val Thr Ala Asn Ser Tyr Asp Ser Ser Thr
           435                 440                 445
   Gly Glu Ile Asp Leu Asn Lys Lys Lys Val Glu Ser Ser Glu Ala Glu
       450                 455                 460
   Tyr Arg Thr Leu Ser Ala Asn Asn Asp Gly Val Tyr Met Pro Leu Gly
   465                 470                 475                 480
   Val Ile Ser Glu Thr Phe Leu Thr Pro Ile Asn Gly Phe Gly Leu Gln
                   485                 490                 495
   Ala Asp Glu Asn Ser Arg Leu Ile Thr Leu Thr Cys Lys Ser Tyr Leu
               500                 505                 510
   Arg Glu Leu Leu Leu Ala Thr Asp Leu Ser Asn Lys Glu Thr Lys Leu
           515                 520                 525
   Ile Val Pro Pro Ile Ser Phe Ile Ser Asn Ile Val Glu Asn Gly Asn
       530                 535                 540
   Leu Glu Gly Glu Asn Leu Glu Pro Trp Ile Ala Asn Asn Lys Asn Ala
   545                 550                 555                 560
   Tyr Val Asp His Thr Gly Gly Ile Asn Gly Thr Lys Val Leu Tyr Val
                   565                 570                 575
   His Lys Asp Gly Glu Phe Ser Gln Phe Val Gly Gly Lys Leu Lys Ser
               580                 585                 590
   Lys Thr Glu Tyr Val Ile Gln Tyr Ile Val Lys Gly Lys Ala Ser Ile
           595                 600                 605
   Tyr Leu Lys Asp Lys Lys Asn Glu Asn Ser Ile Tyr Glu Glu Ile Asn
       610                 615                 620
   Asn Asp Leu Glu Gly Phe Gln Thr Val Thr Lys Arg Phe Ile Thr Gly
   625                 630                 635                 640
   Thr Asp Ser Ser Gly Ile His Leu Ile Phe Thr Ser Gln Asn Gly Glu
                   645                 650                 655
   Gly Ala Phe Gly Gly Asn Phe Ile Ile Ser Glu Ile Arg Thr Ser Glu
               660                 665                 670
   Glu Leu Leu Ser Pro Glu Leu Ile Met Ser Asp Ala Trp Val Gly Ser
           675                 680                 685
   Gln Gly Thr Trp Ile Ser Gly Asn Ser Leu Thr Ile Asn Ser Asn Val
   690 695 700
   Asn Gly Thr Phe Arg Gln Asn Leu Pro Leu Glu Ser Tyr Ser Thr Tyr
   705 710 715 720
   Ser Met Asn Phe Thr Val Asn Gly Phe Gly Lys Val Thr Val Arg Asn
   725 730 735
   Ser Arg Glu Val Leu Phe Glu Lys Ser Tyr Pro Gln Leu Ser Pro Lys
   740 745 750
   Asp Ile Ser Glu Lys Phe Thr Thr Ala Ala Asn Asn Thr Gly Leu Tyr
   755 760 765
   Val Glu Leu Ser Arg Ser Thr Ser Gly Gly Ala Ile Asn Phe Arg Asp
   770 775 780
   Phe Ser Ile Lys
   785
<210> 2
   <211> 619
   <212> PRT
   <213> Artificial Sequence
<220>
   <223> Cry1Ca
<400> 2
   Met Asp Asn Asn Pro Asn Ile Asn Glu Cys Ile Pro Tyr Asn Cys Leu
   1               5                   10                  15
   Ser Asn Pro Glu Glu Val Leu Leu Asp Gly Glu Arg Ile Ser Thr Gly
               20                  25                  30
   Asn Ser Ser Ile Asp Ile Ser Leu Ser Leu Val Gln Phe Leu Val Ser
           35                  40                  45
   Asn Phe Val Pro Gly Gly Gly Phe Leu Val Gly Leu Ile Asp Phe Val
       50                  55                  60
   Trp Gly Ile Val Gly Pro Ser Gln Trp Asp Ala Phe Leu Val Gln Ile
   65                  70                  75                  80
   Glu Gln Leu Ile Asn Glu Arg Ile Ala Glu Phe Ala Arg Asn Ala Ala
                   85                  90                  95
   Ile Ala Asn Leu Glu Gly Leu Gly Asn Asn Phe Asn Ile Tyr Val Glu
               100                 105                 110
   Ala Phe Lys Glu Trp Glu Glu Asp Pro Lys Asn Pro Ala Thr Arg Thr
           115                 120                 125
   Arg Val Ile Asp Arg Phe Arg Ile Leu Asp Gly Leu Leu Glu Arg Asp
       130                 135                 140
   Ile Pro Ser Phe Arg Ile Ser Gly Phe Glu Val Pro Leu Leu Ser Val
   145                 150                 155                 160
   Tyr Ala Gln Ala Ala Asn Leu His Leu Ala Ile Leu Arg Asp Ser Val
                   165                 170                 175
   Ile Phe Gly Glu Arg Trp Gly Leu Thr Thr Ile Asn Val Asn Glu Asn
               180                 185                 190
   Tyr Asn Arg Leu Ile Arg His Ile Asp Glu Tyr Ala Asp His Cys Ala
           195                 200                 205
   Asn Thr Tyr Asn Arg Gly Leu Asn Asn Leu Pro Lys Ser Thr Tyr Gln
       210                 215                 220
   Asp Trp Ile Thr Tyr Asn Arg Leu Arg Arg Asp Leu Thr Leu Thr Val
   225                 230                 235                 240
   Leu Asp Ile Ala Ala Phe Phe Pro Asn Tyr Asp Asn Arg Arg Tyr Pro
                   245                 250                 255
   Ile Gln Pro Val Gly Gln Leu Thr Arg Glu Val Tyr Thr Asp Pro Leu
               260                 265                 270
   Ile Asn Phe Asn Pro Gln Leu Gln Ser Val Ala Gln Leu Pro Thr Phe
           275                 280                 285
   Asn Val Met Glu Asn Ser Ala Ile Arg Asn Pro His Leu Phe Asp Ile
       290                 295                 300
   Leu Asn Asn Leu Thr Ile Phe Thr Asp Trp Phe Ser Val Gly Arg Asn
   305                 310                 315                 320
   Phe Tyr Trp Gly Gly His Arg Val Ile Ser Ser Leu Ile Gly Gly Gly
                   325                 330                 335
   Asn Ile Thr Ser Pro Ile Tyr Gly Arg Glu Ala Asn Gln Glu Pro Pro
               340                 345                 350
   Arg Ser Phe Thr Phe Asn Gly Pro Val Phe Arg Thr Leu Ser Asn Pro
           355                 360                 365
   Thr Leu Arg Leu Leu Gln Gln Pro Trp Pro Ala Pro Pro Phe Asn Leu
   370 375 380
   Arg Gly Val Glu Gly Val Glu Phe Ser Thr Pro Thr Asn Ser Phe Thr
   385 390 395 400
   Tyr Arg Gly Arg Gly Thr Val Asp Ser Leu Thr Glu Leu Pro Pro Glu
   405 410 415
   Asp Asn Ser Val Pro Pro Arg Glu Gly Tyr Ser His Arg Leu Cys His
   420 425 430
   Ala Thr Phe Val Gln Arg Ser Gly Thr Pro Phe Leu Thr Thr Gly Val
   435 440 445
   Val Phe Ser Trp Thr His Arg Ser Ala Thr Leu Thr Asn Thr Ile Asp
   450 455 460
   Pro Glu Arg Ile Asn Gln Ile Pro Leu Val Lys Gly Phe Arg Val Trp
   465 470 475 480
   Gly Gly Thr Ser Val Ile Thr Gly Pro Gly Phe Thr Gly Gly Asp Ile
   485 490 495
   Leu Arg Arg Asn Thr Phe Gly Asp Phe Val Ser Leu Gln Val Asn Ile
   500 505 510
   Asn Ser Pro Ile Thr Gln Arg Tyr Arg Leu Arg Phe Arg Tyr Ala Ser
   515 520 525
   Ser Arg Asp Ala Arg Val Ile Val Leu Thr Gly Ala Ala Ser Thr Gly
   530 535 540
   Val Gly Gly Gln Val Ser Val Asn Met Pro Leu Gln Lys Thr Met Glu
   545 550 555 560
   Ile Gly Glu Asn Leu Thr Ser Arg Thr Phe Arg Tyr Thr Asp Phe Ser
   565 570 575
   Asn Pro Phe Ser Phe Arg Ala Asn Pro Asp Ile Ile Gly Ile Ser Glu
   580 585 590
   Gln Pro Leu Phe Gly Ala Gly Ser Ile Ser Ser Gly Glu Leu Tyr Ile
   595 600 605
   Asp Lys Ile Glu Ile Ile Leu Ala Asp Ala Thr
   610 615

## Description

### Background of the Invention

Humans grow corn for food and energy applications. Humans also grow many other crops, including soybeans and cotton. Insects eat and damage plants and thereby undermine these human efforts. Billions of dollars are spent each year to control insect pests and additional billions are lost to the damage they inflict. Synthetic organic chemical insecticides have been the primary tools used to control insect pests but biological insecticides, such as the insecticidal proteins derived from *Bacillus thuringiensis* (*Bt*), have played an important role in some areas. The ability to produce insect-resistant plants through transformation with *Bt* insecticidal protein genes has revolutionized modern agriculture and heightened the importance and value of insecticidal proteins and their genes.

Several *Bt* proteins have been used to create the insect-resistant transgenic plants that have been successfully registered and commercialized to date. These include Cry1Ab, Cry1Ac, Cry1F and Cry3Bb in corn, Cry1Ac and Cry2Ab in cotton, and Cry3A in potato.

The commercial products expressing these proteins express a single protein except in cases where the combined insecticidal spectrum of 2 proteins is desired (*e.g.,* Cry1Ab and Cry3Bb in corn combined to provide resistance to lepidopteran pests and rootworm, respectively) or where the independent action of the proteins makes them useful as a tool for delaying the development of resistance in susceptible insect populations (*e.g.,* Cry1Ac and Cry2Ab in cotton combined to provide resistance management for tobacco budworm). *See also* U.S. Patent Application Publication No. 2009/0313717, which relates to a Cry2 protein plus a Vip3Aa, Cry1F, or Cry1A for control of *Helicoverpa zea* or *armigerain.* WO 2009/132850 relates to Cry1F or Cry1A and Vip3Aa for controlling *Spodoptera frugiperda.* U.S. Patent Application Publication No. 2008/0311096 relates in part to Cry1Ab for controlling Cry1F-resistant ECB.

That is, some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The proteins selected for use in an insect resistant management (IRM) stack need to exert their insecticidal effect independently so that resistance developed to one protein does not confer resistance to the second protein (*i.e.,* there is not cross resistance to the proteins). If, for example, a pest population that is resistant to "Protein A" is sensitive to "Protein B", one would conclude that there is not cross resistance and that a combination of Protein A and Protein B would be effective in delaying resistance to Protein A alone.

In the absence of resistant insect populations, assessments can be made based on other characteristics presumed to be related to mechanism of action and cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not be simultaneously resistant to those two toxins.

For example, CrylFa protein is useful in controlling many lepidopteran pests species including the European corn borer (ECB; *Ostrinia nubilalis* (Hübner)) and the fall armyworm (FAW; *Spodoptera frugiperda*), and is active against the sugarcane borer (SCB; *Diatraea saccharalis)*. The CrylFa protein, as produced in transgenic corn plants containing event TC1507, is responsible for an industry-leading insect resistance trait for FAW control. CrylFa is further deployed in the Herculex^{®}, SmartStax™, and WideStrike™ products.

Additional *Cry* toxins are listed at the website of the official *B.t.* nomenclature committee (Crickmore *et al*.; lifesci.sussex.ac.uk/home/Neil_Crickmore/Bt/). There are currently nearly 60 main groups of "Cry" toxins (Cry1-Cry59), with additional Cyt toxins and VIP toxins and the like. Many of each numeric group have capital-letter subgroups, and the capital letter subgroups have lower-cased letter sub-subgroups. (Cry1 has A-L, and Cry1A has a-i, for example).

### Brief Summary of the Invention

The subject invention relates in part to the use of a Vip3Ab protein in combination with a Cry1Ca protein and a Cry1Fa or Cry1Da protein. Plants (and acreage planted with such plants) that produce these proteins are included within the scope of the subject invention.

These particular triple stacks would, according to the subject invention, advantageously and surprisingly provide three sites of action against FAW. This can help to reduce or eliminate the requirement for refuge acreage.

The subject invention relates in part to the surprising discovery that Vip3Ab does not compete with Cry1Ca for binding sites in the gut of fall armyworm (*Spodoptera frugiperda*; FAW).

The subject invention also relates in part to triple stacks or "pyramids" with further toxins. In some preferred pyramid embodiments, the combination of the selected toxins provides non-cross-resistant action against FAW.

Additional toxins/genes can also be added according to the subject invention adding two additional proteins to the triple stack wherein the two added proteins target ECB, would provide three sites of action against FAW, and three sites of action against ECB. These two added proteins (the fourth and fifth proteins) could be selected from the group consisting of Cry2A, Cry1I, DIG-3, and Cry1Ab. This would result in a five-protein stack having three sites of action against two insects (ECB and FAW).

### DETAILED DESCRIPTION OF THE INVENTION

The subject invention relates in part to the surprising discovery that Vip3Ab and Cry1Ca do not compete for binding with each other in the gut of fall armyworms (FAW; *Spodoptera frugiperda).* The three subject proteins Vip3Ab, Cry1Ca and one of CryFa or Cry1Da can be used in transgenic corn (and other plants; e.g., cotton and soybeans, for example) to delay or prevent FAW from developing resistance to one of these proteins alone. The subject proteins can be effective at protecting plants (such as maize plants and/or soybean plants) from damage by Cry-resistant fall armyworm. That is, one use of the subject invention is to protect corn and other economically important plant species from damage and yield loss caused by fall armyworm populations that could develop resistance to the subject proteins.

The subject invention thus teaches an insect resistant management (IRM) stack comprising the subject proteins to prevent or mitigate the development of resistance by FAW to either or both of these proteins.

Disclosed are compositions for controlling lepidopteran pests comprising cells that produce a Vip3Ab insecticidal protein and a Cry1Ca insecticidal protein.

The invention further comprises a plant cell to produce the subject proteins. The subject polynucleotide(s) are preferably in a genetic construct under control of a non*-Bacillus-thuringiensis* promoter(s). The subject polynucleotides can comprise codon usage for enhanced expression in a plant.

It is additionally intended that the invention provides a method of controlling FAW comprising contacting said pests or the environment of said pests with an effective amount of a composition that contains the subject proteins.

An embodiment of the invention comprises a maize plant comprising a plant-expressible genes encoding the subject proteins, and seed of such a plant.

A further embodiment of the invention comprises a maize plant wherein a plant-expressible genes encoding the subject proteins have been introgressed into said maize plant, and seed of such a plant.

As described in the Examples, competitive receptor binding studies using radiolabeled Cry1Ca protein show that the Cry1Ca protein does not compete for binding in FAW tissues to which Vip3Ab binds. These results also indicate that the combination of Vip3Ab and Cry1Ca proteins can be an effective means to mitigate the development of resistance in FAW populations to either of these proteins. Thus, based in part on the data described herein, it is thought that co-production (stacking) of the Cry1Ca and Vip3Ab proteins can be used to produce a high dose IRM stack for FAW.

Other proteins can be added. For example, the subject invention also relates in part to stacks or "pyramids" of more toxins, with the subject proteins being the base. In some preferred pyramid embodiments, the selected toxins have three separate sites of action against FAW. By "separate sites of action," it is meant any of the given proteins do not cause cross-resistance with each other. These particular stacks would, according to the subject invention, advantageously and surprisingly provide three sites of action against FAW. This can help to reduce or eliminate the requirement for refuge acreage.

Related to some specific embodiments of the subject invention, we showed that a FAW population resistant to the insecticidal activity of the Cry1Fa protein is not resistant to the insecticidal activity of the Vip3Ab protein or to the insecticidal activity of the Cry1Ca protein. We demonstrated that Cry1Ca does not compete for the binding sites with Cry1Fa and that Vip3Ab does not compete for the binding sites with CrylFa in the gut of FAW. *See* USSN 61/284,281 (filed December 16, 2009) regarding CrylFa and Cry1Ca, and concurrently filed PCT application entitled "COMBINED USE OF Vip3Ab AND CRY1Fa FOR MANAGEMENT OF RESISTANT INSECTS ")

Thus, the pairs of toxins CrylFa plus Vip3Ab and CrylFa plus Cry1Ca provide non-cross-resistant action against FAW. The inability of Vip3Ab1 to compete for the binding of Cry1Ca in the gut of FAW demonstrates that these three protein toxins (Cry1Fa, Vip3Ab, and CrylCa) represent a triple-stack pyramid of Cry toxins that provide three separate target site interactions within the gut of FAW. These particular triple stacks would, according to the subject invention, advantageously and surprisingly provide non-cross-resistant action against FAW. Furthermore, by the demonstration that these three proteins do not compete with each other, one skilled in the art will recognize that this can help to reduce or eliminate the requirement for refuge acreage. As with the benefit of this disclosure, plants expressing the triple combination of Cry1Fa, Vip3Ab and Cry1Ca, will be useful in delaying or preventing the development of resistance in FAW to the individual or combination of these proteins.

Additional toxins/genes can also be added according to the subject invention. For example, if Cry1Fa is stacked with Vip3Ab and Cry1Ca, adding two additional proteins to this triple stack wherein the two added proteins target ECB, would provide three sites of action against FAW, and three sites of action against ECB. These two added proteins (the fourth and fifth proteins) could be selected from the group consisting of Cry2A, Cry1I, DIG-3 (see U.S. Patent Application Serial No. 61/284,278 (filed December 16, 2009) and US 2010 00269223), and Cry1Ab. This would result in a five-protein stack having three sites of action against two insects (ECB and FAW)

Thus, one deployment option is to use the subject proteins to mitigate the development of resistance in FAW to any of these toxins. Accordingly, the subject invention also relates to triple stacks or "pyramids" of three (or more) toxins. In some preferred pyramid embodiments, the selected toxins have three separate sites of action against FAW.

Included among deployment options of the subject invention would be to use at least, the subject proteins in crop-growing regions where FAW can develop resistant populations.

With Cry1Fa being active against FAW and ECB, Vip3Ab plus Cry1Ca plus CrylFa would, according to the subject invention, advantageously and surprisingly provide three sites of action against FAW. This can help to reduce or eliminate the requirement for refuge acreage.

Cry1Fa is deployed in the Herculex^{®}, SmartStax™, and WidesStrike™ products. The genes Vip3Ab and Cry1Ca could be combined into, for example, a Cry1Ca product such as Herculex^{®}, SmartStax™, and WideStrike™. Accordingly, the subject proteins could be significant in reducing the selection pressure on these and other proteins. The subject proteins could thus be used for corn and other plants (cotton and soybeans, for example).

As discussed above, additional toxins/genes can also be added according to the subject invention. For the use of CrylE (for controlling FAW), *see* U.S. Patent Application Serial No. 61/284,278 (filed December 16, 2009).

Plants that produce any of the subject combinations of proteins are included within the scope of the subject invention. Additional toxins/genes can also be added, but the particular stacks discussed above advantageously and surprisingly provide multiple sites of action against FAW and/or ECB. This can help to reduce or eliminate the requirement for refuge acreage.

GENBANK can also be used to obtain the sequences for any of the genes and proteins disclosed or mentioned herein. *See* Appendix A, below. Relevant sequences are also available in patents. For example, U.S. Patent No. 5,188,960 and U.S. Patent No. 5,827,514 describe CrylFa core toxin containing proteins suitable for use in carrying out the present invention. U.S. Patent No. 6,218,188 describes plant-optimized DNA sequences encoding Cry1Fa core toxin-containing proteins that are suitable for use in the present invention. USSN 61/284,275 (filed December 16, 2009) provides some truncated Cry1Ca proteins that can be used according to the subject invention.

Combinations of proteins described herein can be used to control lepidopteran pests. Adult lepidopterans, for example, butterflies and moths, primarily feed on flower nectar and are a significant effector of pollination. Nearly all lepidopteran larvae, *i.e*., caterpillars, feed on plants, and many are serious pests. Caterpillars feed on or inside foliage or on the roots or stem of a plant, depriving the plant of nutrients and often destroying the plant's physical support structure. Additionally, caterpillars feed on fruit, fabrics, and stored grains and flours, ruining these products for sale or severely diminishing their value. As used herein, reference to lepidopteran pests refers to various life stages of the pest, including larval stages.

Some chimeric toxins of the subject invention comprise a full N-terminal core toxin portion of a *Bt* toxin and, at some point past the end of the core toxin portion, the protein has a transition to a heterologous protoxin sequence. The N-terminal, insecticidally active, toxin portion of a *Bt* toxin is referred to as the "core" toxin. The transition from the core toxin segment to the heterologous protoxin segment can occur at approximately the toxin/protoxin junction or, in the alternative, a portion of the native protoxin (extending past the core toxin portion) can be retained, with the transition to the heterologous protoxin portion occurring downstream.

As an example, one chimeric toxin of the subject invention, is a full core toxin portion of Cry1Ca (roughly the first 600 amino acids) and/or a heterologous protoxin (the remaining amino acids to the C-terminus). In one preferred embodiment, the portion of a chimeric toxin comprising the protoxin is derived from a Cry1Ab protein toxin. In a preferred embodiment, the portion of a chimeric toxin comprising the protoxin is derived from a Cry1Ab protein toxin.

A person skilled in this art will appreciate that *Bt* toxins, even within a certain class such as Cry1Ca, will vary to some extent in length and the precise location of the transition from core toxin portion to protoxin portion. Typically, the Cry1Ca toxins are about 1150 to about 1200 amino acids in length. The transition from core toxin portion to protoxin portion will typically occur at between about 50% to about 60% of the full length toxin. The chimeric toxin of the subject invention will include the full expanse of this N-terminal core toxin portion. Thus, the chimeric toxin will comprise at least about 50% of the full length of the Cry1 *Bt* toxin protein. This will typically be at least about 590 amino acids. With regard to the protoxin portion, the full expanse of the Cry1Ab protoxin portion extends from the end of the core toxin portion to the C-terminus of the molecule.

Genes and toxins. The genes and toxins useful according to the subject invention include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

As used herein, the boundaries represent approximately 95% (Vip3Ab 's and Cry1Ca's), 78% (Vip3Ab's and Cry1C's), and 45% (Cry1's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. These cut offs can also be applied to the core toxins only.

It should be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments and equivalents which retain the pesticidal activity of the exemplified toxins would be within the scope of the subject invention. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. These variant DNA sequences are within the scope of the subject invention. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Variant toxins. Certain toxins of the subject invention have been specifically exemplified herein it should be readily apparent that the subject invention comprises variant or equivalent toxins (and nucleotide sequences coding for equivalent toxins) having the same or similar pesticidal activity of the exemplified toxin. Equivalent toxins will have amino acid homology with an exemplified toxin. This amino acid homology will typically be greater than 75%, preferably be greater than 90%, and most preferably be greater than 95%. The amino acid homology will be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Below is a listing of examples of amino acids belonging to each class.

| **Class of Amino Acid** | **Examples of Amino Acids** |
|---|---|
| Nonpolar | Ala, Val, Leu, Ile, Pro, Met, Phe, Trp |
| Uncharged Polar | Gly, Scr, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin.

Recombinant hosts. The genes encoding the toxins of the subject invention can be introduced into a wide variety of microbial or plant hosts. Expression of the toxin gene results, directly or indirectly, in the intracellular production and maintenance of the pesticide. Conjugal transfer and recombinant transfer can be used to create a *Bt* strain that expresses both toxins of the subject invention. Other host organisms may also be transformed with one two or three of the toxin genes then used to accomplish the synergistic effect. With suitable microbial hosts, *e.g*., *Pseudomonas*, the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest.

Where the *Bt* toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, *e.g*., genera *Pseudomonas, Erwinia, Serratia, Klebsiella, Xanthomonas, Streptomyces, Rhizobium, Rhodopseudomonas, Methylophilius, Agrobactenum, Acetobacter Lactobacillus, Arthrobacter, Azotobacter, Leuconostoc,* and *Alcaligenes;* fungi, particularly yeast, *e.g.,* genera *Saccharomyces, Cryptococcus, Kluyveromyces, Sporobolomyces, Rhodotorula,* and *Aureobasidium.* Of particular interest are such phytosphere bacterial species as *Pseudomonas syringae*, *Pseudomonas fluorescens, Serratia marcescens, Acetobacter xylinum, Agrobactenium tumefaciens, Rhodopseudomonas spheroides, Xanthomonas campestris, Rhizobium melioti, Alcaligenes entrophus,* and *Azotobacter vinlandii;* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans.* Of particular interest are the pigmented microorganisms.

A wide variety of methods is available for introducing a *Bt* gene encoding a toxin into a microorganism host under conditions which allow for stable maintenance and expression of the gene. These methods are well known to those skilled in the art and are described, for example, in U.S. Patent No. 5,135,867.

Treatment of cells. *Bacillus thuringiensis* or recombinant cells expressing the *Bt* toxins can be treated to prolong the toxin activity and stabilize the cell. The pesticide microcapsule that is formed comprises the *Bt* toxin or toxins within a cellular structure that has been stabilized and will protect the toxin when the microcapsule is applied to the environment of the target pest. Suitable host cells may include either prokaryotes or eukaryotes, normally being limited to those cells which do not produce substances toxic to higher organisms, such as mammals. However, organisms which produce substances toxic to higher organisms could be used, where the toxic substances are unstable or the level of application sufficiently low as to avoid any possibility of toxicity to a mammalian host. As hosts, of particular interest will be the prokaryotes and the lower eukaryotes, such as fungi.

The cell will usually be intact and be substantially in the proliferative form when treated, rather than in a spore form, although in some instances spores may be employed.

Treatment of the microbial cell, e.g., a microbe containing the B.t. toxin gene or genes, can be by chemical or physical means, or by a combination of chemical and/or physical means, so long as the technique does not deleteriously affect the properties of the toxin, nor diminish the cellular capability of protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as glutaraldehyde; anti-infectives, such as zephiran chloride and cetylpyridinium chloride; alcohols, such as isopropyl and ethanol; various histologic fixatives, such as Lugol iodine, Bouin's fixative, various acids and Helly's fixative (See: Humason, Gretchen L., Animal Tissue Techniques, W. H. Freeman and Company, 1967); or a combination of physical (heat) and chemical agents that preserve and prolong the activity of the toxin produced in the cell when the cell is administered to the host environment. Examples of physical means are short wavelength radiation such as gamma-radiation and X-radiation, freezing, UV irradiation, lyophilization, and the like. Methods for treatment of microbial cells are disclosed in U.S. Pat. Nos. 4,695,455 and 4,695,462.

The cells generally will have enhanced structural stability which will enhance resistance to environmental conditions. Where the pesticide is in a proform, the method of cell treatment should be selected so as not to inhibit processing of the proform to the mature form of the pesticide by the target pest pathogen. For example, formaldehyde will crosslink proteins and could inhibit processing of the proform of a polypeptide pesticide. The method of treatment should retain at least a substantial portion of the bio-availability or bioactivity of the toxin.

Characteristics of particular interest in selecting a host cell for purposes of production include ease of introducing the B.t. gene or genes into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

Growth of cells. The cellular host containing the B.t. insecticidal gene or genes may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells producing the toxins of the invention can be cultured using standard art media and fermentation techniques. Upon completion of the fermentation cycle the bacteria can be harvested by first separating the B.t. spores and crystals from the fermentation broth by means well known in the art. The recovered B.t. spores and crystals can be formulated into a wettable powder, liquid concentrate, granules or other formulations by the addition of surfactants, dispersants, inert carriers, and other components to facilitate handling and application for particular target pests. These formulations and application procedures are all well known in the art.

Formulations. Formulated bait granules containing an attractant and spores, crystals, and toxins of the B.t. isolates, or recombinant microbes comprising the genes obtainable from the B.t. isolates disclosed herein, can be applied to the soil. Formulated product can also be applied as a seed-coating or root treatment or total plant treatment at later stages of the crop cycle. Plant and soil treatments of B.t. cells may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

As would be appreciated by a person skilled in the art, the pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the lepidopteran pest, e.g., foliage or soil, by spraying, dusting, sprinkling, or the like.

Plant transformation. A preferred recombinant host for production of the insecticidal proteins of the subject invention is a transformed plant. Genes encoding *Bt* toxin proteins, as disclosed herein, can be inserted into plant cells using a variety of techniques which are well known in the art. For example, a large number of cloning vectors comprising a replication system in *Escherichia coli* and a marker that permits selection of the transformed cells are available for preparation for the insertion of foreign genes into higher plants. The vectors comprise, for example, pBR322, pUC series, M13mp series, pACYC184, *inter alia.* Accordingly, the DNA fragment having the sequence encoding the *Bt* toxin protein can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516, Lee and Gelvin (2008), Hoekema (1985), Fraley *et al*., (1986), and An *et al*., (1985), and is well established in the art.

Once the inserted DNA has been integrated in the plant genome, it is relatively stable. The transformation vector normally contains a selectable marker that confers on the transformed plant cells resistance to a biocide or an antibiotic, such as Bialaphos, Kanamycin, G418, Bleomycin, or Hygromycin, *inter alia.* The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, biolistics (microparticle bombardment), or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E. coli* and in Agrobacteria. They comprise a selection marker gene and a linker or polylinker which are framed by the Right and Left T-DNA border regions. They can be transformed directly into Agrobacteria (Holsters *et al*., 1978). The *Agrobacterium* used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

In a preferred embodiment of the subject invention, plants will be transformed with genes wherein the codon usage has been optimized for plants. See, for example, U.S. Patent No. 5,380,831. While some truncated toxins are exemplified herein, it is well-known in the *Bt* art that 130 kDa-type (full-length) toxins have an N-terminal half that is the core toxin, and a C-terminal half that is the protoxin "tail." Thus, appropriate "tails" can be used with truncated / core toxins of the subject invention. *See e.g.* U.S. Patent No. 6,218,188 and U.S. Patent No. 6,673,990. In addition, methods for creating synthetic *Bt* genes for use in plants are known in the art (Stewart and Burgin, 2007). One non-limiting example of a preferred transformed plant is a fertile maize plant comprising a plant expressible genes encoding the subject proteins.

Transfer (or introgression) of the subject protein determined trait(s) into inbred maize lines can be achieved by recurrent selection breeding, for example by backcrossing. In this case, a desired recurrent parent is first crossed to a donor inbred (the non-recurrent parent) that carries the appropriate gene(s) for the traits. The progeny of this cross is then mated back to the recurrent parent followed by selection in the resultant progeny for the desired trait(s) to be transferred from the non-recurrent parent. After three, preferably four, more preferably five or more generations ofbackcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush *et al*., for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppdl/biopesticides/pips/bt_corn_refuge_2006.htm) publishes the following requirements for providing non-transgenic (*i.e., non-B.t.)* refuges (a section of non-Bt crops / corn) for use with transgenic crops producing a single Bt protein active against target pests.
"The specific structured requirements for corn borer-protected Bt (CrylAb or Cry1F) corn products are as follows:
Structured refuges: 20% non-Lepidopteran Bt corn refuge in Corn Belt;
   50% non-Lepidopteran Bt refuge in Cotton Belt
Blocks
   Internal (*i.e*., within the Bt field)
   External (*i.e.*, separate fields within ½ mile (¼ mile if possible) of the
   Bt field to maximize random mating)
In-field Strips
   Strips must be at least 4 rows wide (preferably 6 rows) to reduce
   the effects of larval movement"

In addition, the National Corn Growers Association, on their website:
(ncga.com/insect-resistance-management-fact-sheet-bt-corn)

also provides similar guidance regarding the refuge requirements. For example:
"Requirements of the Corn Borer IRM:
   - Plant at least 20% of your corn acres to refuge hybrids
   - In cotton producing regions, refuge must be 50%
   - Must be planted within 1/2 mile of the refuge hybrids
   - Refuge can be planted as strips within the Bt field; the refuge strips must be at least 4 rows wide
   - Refuge may be treated with conventional pesticides only if economic thresholds are reached for target insect
   - Bt-based sprayable insecticides cannot be used on the refuge corn
   - Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S. Patent No. 6,551,962.

The above percentages, or similar refuge ratios, can be used for the subject triple stacks or pyramids. For triple stacks with three sites of action against a single target pest, a goal would be zero refuge (or less than 5% refuge, for example). This is particularly true for commercial acreage - of over 10 acres for example.

Unless specifically indicated or implied, the terms "a", "an", and "the" signify "at least one" as used herein.

Following are examples that illustrate procedures for practicing the invention. These examples should not be construed as limiting. All percentages are by weight and all solvent mixture proportions are by volume unless otherwise noted. All temperatures are in degrees Celsius.

### EXAMPLES

### Example 1- Production and trypsin processin of Vip3Ab and Cry1Ca proteins.

The genes encoding the Cry1Ca and Vip3Ab1 pro toxins were expressed in *Pseudomonas fluorescens* expression strains and the full length proteins isolated as insoluble inclusion bodies. The washed inclusion bodies were solubilized by stirring at 37 °C in buffer containing 20 mM CAPS buffer, pH 11, + 10 mM DDT, + 0.1% 2-mercaptoethanol, for 2 hrs. The solution was centrifuged at 27,000 x g for 10 min. at 37 °C and the supernatant treated with 0.5% (w/v) TCPK treated trypsin (Sigma). This solution was incubated with mixing for an additional 1 hr. at room temperature, filtered, then loaded onto a Pharmacia Mono Q 1010 column equilibrated with 20 mM CAPS pH 10.5. After washing the loaded column with 2 column volumes of buffer, the truncated toxin was eluted using a linear gradient of 0 to 0.5 M NaCl in 20 mM CAPS in 15 column volumes at a flow rate of 1.0 ml/min. Purified trypsin truncated Cry proteins eluted at about 0.2-0.3 M NaCl. The purity of the proteins was checked by SDS PAGE and with visualization using Coomassie brilliant blue dye. In some cases, the combined fractions of the purified toxin were concentrated and loaded onto a Superose 6 column (1.6 cm dia., 60 cm long), and further purified by size exclusion chromatography. Fractions comprising a single peak of the monomeric molecular weight were combined, and concentrated, resulting in a preparation more than 95% homogeneous for a protein having a molecular weight of about 60,000 kDa.

Processing of Vip3Ab1 was achieved in a similar manner starting with the purified full length 85 kDa protein (DIG-307). The protein (12 mg) was dialyzed into 50 mM sodium phosphate buffer, pH 8.4, then processed by adding 1 mg of solid trypsin and incubating for 1 hrs. at room temperature. The solution was loaded onto a MonoQ anion exchange column (1 cm dia., 10 cm. long), and eluted with a linear gradient of NaCl from 0 to 500 mM in 20 mM sodium phosphate buffer, pH 8.4 over 7 column volumes. Elution of the protein was monitored by SDS-PAGE. The major processed band had a molecular weight of 65 kDa, as determined by SDS-PAGE using molecular weight standards for comparison.

### Example 2 - Iodination of Cry1Ca core toxin protein

Previous work indicated that Cry1Ca was very difficult to radiolabel using traditional methods, although in a select few cases it would radiolabel and function well in a receptor binding assay. We decided to radiolabel Cry1Ca using ¹²⁵I radiolabeled fluorescein-5-maleimide, which is a method that has worked to actively radiolabel Cry1Fa (Prov. 69919). Iodination of fluroescein-5- malemide and subsequent conjugation of this radiolabeled chemical with Cry1Ca results in cysteine specific radiolabeling of the protein. Such labeling procedure is thus highly specific in the residues that are labeled. The Cry1Ca core toxin segment (residues 29-619) contains two cysteine amino acid residues, at positions 210 and 438. Palmer *et al.* (1997) demonstrated that the phenyl rings of fluorescein-5-maleimide can be radio-iodinated and then reacted with proteins that contain sulfhydryl groups (*e.g.* as provided by free cysteine residues), resulting in alkylation of the free cysteines in the protein, and thus providing a radioactively labeled protein. The trypsin-truncated Cry1Ca core toxin contains two cysteine residues and thus provides a substrate for alkylation and radiolabeling of the protein at these two (specific) sites.

Fluorescein-5-maleimide (F5-M) was dissolved to 10 mM in DMSO (Dimethyl Sulfoxide), then diluted to 1 mM in phosphate buffered saline (PBS; 20 mM sodium phosphate, 0.15 M NaCl, pH7.5), as determined by the molar extinction coefficient of F 5-M (68,000 M⁻¹cm⁻¹). To a 100 µL solution of PBS containing two Pierce Iodination Beads (Thermo Fisher Scientific), 1.0 mCi of Na¹²⁵I was added behind lead shielding. The solution was allowed to mix at room temperature for 5 min, then 10 µL of the 1 mM F 5-M solution were added. After reacting for 10 min, the solution was removed from the iodination reaction by pipetting and 2 µg of highly purified trypsin-truncated Cry1Ca core toxin protein in PBS were added to the solution. The protein was incubated at 4° with the iodinated F 5-M solution for 48 hrs, when the reaction was terminated by adding β-mercaptoethanol to 14 mM final concentration. The reaction mixture was added to a Zebra™ spin column (Invitrogen) equilibrated in 20 mM CAPS, 150 mM KCl, pH9, and centrifuged at 1500 x g for 2 min to separate non-reacted iodinated dye from the protein. The ¹²⁵I radiolabeled fluorescein-Cry1Ca core toxin protein was counted in a gamma counter to determine its specific radioactivity, assuming 80% recovery of the input toxin protein.

The specific activity of the radiolabeled Cry1Ca core toxin protein was approximately 6.8 µCi/µg protein. The radiolabeled protein was also characterized by SDS-PAGE and visualized by phosphor-imaging to validate that the radioactivity measured was covalently associated with the Cry1Ca core toxin protein. Coomassie stained SDS-PAGE gels were imaged by wrapping them in Mylar™ film (12 µm thick), and exposing them under a Molecular Dynamics (Sunnyvale, CA) storage phosphor screen (35 cm x 43 cm) for 1 hour. The plates were developed using a Molecular Dynamics Storm 820 phosphor-imager and the image analyzed using ImageQuant™ software. Some radioactivity was detectable in the gel region well below the Cry1Ca core toxin protein band *(i.e.* fragments smaller than the Cry1Ca core toxin protein at about 10 kDa in size and lower). These radioactive contaminants likely represent small peptides probably associated in the truncated Cry1Ca protein due to the action of the trypsin used to cleave the protein to its core structure.

### Example 3 - Competitive binding assays to BBMVs from S. frugiperda with core toxin proteins of Cry1Ca and Vip3Ab.

Homologous and heterologous competition binding assays were conducted using 150 µg/mL BBMV protein and 2 nM of the 125I-radiolabeled Cry1Ca core toxin protein. Concentrations of the homologous competitive non-radiolabeled Cry1Ca core toxin protein added to the reaction mixture was 0.1, 1, 10, 100, and 1000 nM. The heterologous trypsin truncated Vip3Ab protein was tested at 10 and 1,000 nM and the proteins were added at the same time as the radioactive Cry1Ca core toxin protein to assure true binding competition. Incubations were carried out for 1 hr at 28° and the amount of 125I-labeled Cry1Ca core toxin protein unbound to the BBMV's (that is, not bound to an insect receptor protein) is separated from bound protein by centrifugation of the BBMV mixture at 16,000 x g for 8 min, and removing the supernatant from the resulting pellet. The pellet is washed three times with ice cold binding buffer (PBS; 11.9 mM Na₂HPO₄, 137 mM NaCl, 2.7 mM KCl, pH7.4 plus 0.1% bovine serum albumin; Sigma-Aldrich, St. Louis, MO) to completely remove any remaining unbound ¹²⁵I labeled Cry1Ca. The bottom the centrifuge tube was cut out and the protein pellet contained within this section placed in a 13 x 100 mm glass culture tube and counted in a gamma counter for 10 minutes to obtain the amount of bound radioactivity contained the pellet fraction. The amount of radioactivity in the bound protein fraction provides an indication of the amount of Cry protein bound to the insect receptor (total binding). Non-specific binding was represented by the counts obtained in the pellet in the presence of 1,000 nM of non-radiolabeled Cry1Ca core toxin protein. The amount of radiolabeled Cry1Ca specifically bound to the BBMV (specific binding) was measured by subtracting the level of total binding from non specific binding. One hundred percent total binding was considered to be the amount of binding in the absence of any competitor Cry1Fa core toxin protein. The data is expressed as percent of specific bound ¹²⁵I Cry1Ca versus concentration of competitive unlabeled ligand.

### Example 4 - Summary of Results

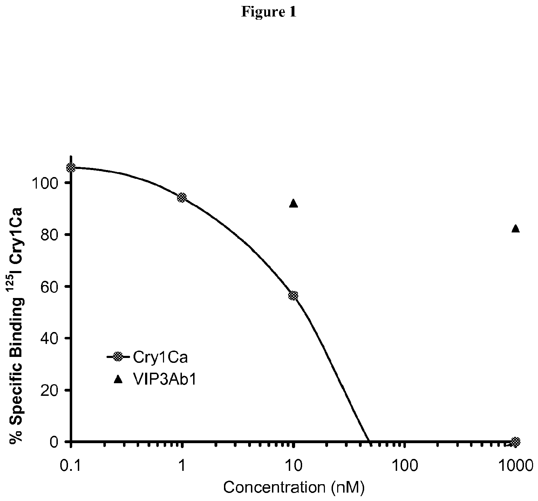

The results (**Figure 1**) show that the homologous unlabeled Cry1Ca protein effectively displaced the radiolabeled Cry1Ca core toxin protein from specifically binding to the BBMV proteins in a dose dependent manner. Vip3Ab did not displace bound 125I-labeled Cry1Ca core toxin protein from its receptor protein(s) at either of the concentrations shown (10 or 1,000 nM). The highest concentration of Vip3Ab tested (1,000 nM) represents 500-fold greater concentration than the radiolabeled Cry1Ca used in the assay, demonstrating that Vip3Ab does not effectively compete with the binding of radiolabeled Cry1Ca in *S. frugiperda* BBMV.

Figure 1 is a dose response curve for the displacement of ¹²⁵I radiolabeled fluorescein-5-maleimide trypsin-truncated Cry1Ca in BBMV's from *S. frugiperda* (FAW) larvae. The figure shows the ability of non-labled Cry1Ca (●) to displace the labeled Cry1Ca in a dose dependent manner in the range from 0.1 to 1,000 nM. The chart plots the percent of specifically bound labeled Cry1Ca (total bound minus non-specific bound) versus the concentration of the non-radiolabeled ligands added. The inability of non radiolabeled Vip3Ab1 (▲) at 10 and 1,000 nM to displace the specifically bound radiolabeled Cry1Ca is shown.

### Reference List

Heckel,D.G., Gahan,L.J., Baxter,S.W., Zhao,J.Z., Shelton,A.M., Gould,F., and Tabashnik,B.E. (2007). The diversity of Bt resistance genes in species of Lepidoptera. J Invertebr Pathol 95, 192-197.
Luo,K., Banks,D., and Adang,M.J. (1999). Toxicity, binding, and permeability analyses of four bacillus thuringiensis cry1 delta-endotoxins using brush border membrane vesicles of spodoptera exigua and spodoptera frugiperda. Appl. Environ. Microbiol. 65, 457-464.
Palmer, M., Buchkremer, M, Valeva, A, and Bhakdi, S. Cysteine-specific radioiodination of proteins with fluorescein maleimide. Analytical Biochemistry 253, 175-179. 1997. Ref Type: Journal (Full)
Sambrook,J. and Russell,D.W. (2001). Molecular Cloning: A Laboratory Manual. Cold Spring Harbor Laboratory).
Schlenz, M. L., Babcock, J. M., and Storer, N. P. Response of Cry1F-resistant and Susceptible European Corn Borer and Fall Armyworm Colonies to Cry 1A. 105 and Cry12Ab2. DAI 0830, 2008. Indianapolis, Dow AgroSciences. Derbi Report.
Sheets, J. J. and Storer, N. P. Analysis of Cry1Ac Binding to Proteins in Brush Border Membrane Vesicles of Corn Earworm Larvae (Heleothis zea). Interactions with Cry1F Proteins and Its Implication for Resistance in the Field. DAI-0417, 1-26. 2001. Indianapolis, Dow AgroSciences.
Tabashnik,B.E., Liu,Y.B., Finson,N., Masson,L., and Heckel,D.G. (1997). One gene in diamondback moth confers resistance to four Bacillus thuringiensis toxins. Proc. Natl. Acad. Sci. U. S. A 94, 1640-1644.
Tabashnik,B.E., Malvar,T., Liu,Y.B., Finson,N., Borthakur,D., Shin,B.S., Park,S.H., Masson,L., de Maagd,R.A., and Bosch,D. (1996). Cross-resistance of the diamondback moth indicates altered interactions with domain II of Bacillus thuringiensis toxins. Appl. Environ. Microbiol. 62, 2839-2844.
Tabashnik,B.E., Roush,R.T., Earle,E.D., and Shelton,A.M. (2000). Resistance to Bt toxins. Science 287, 42.
Wolfersberger,M.G. (1993). Preparation and partial characterization of amino acid transporting brush border membrane vesicles from the larval midgut of the gypsy moth (Lymantria dispar). Arch. Insect Biochem. Physiol 24, 139-147.
Xu,X., Yu,L., and Wu,Y. (2005). Disruption of a cadherin gene associated with resistance to Cry1Ac {delta}-endotoxin of Bacillus thuringiensis in Helicoverpa armigera. Appl Environ Microbiol 71, 948-954.

### Appendix A

**List of delta-endotoxins - from Crickmore et al. website (cited in application) Accession Number is to NCBI entry**

| **Name** | **Acc No.** | **Authors** | **Year** | **Source Strain** | **Comment** |
|---|---|---|---|---|---|
| Cry1Aa1 | AAA22353 | Schnepf et al | 1985 | Bt kurstaki HD1 | |
| Cry1Aa2 | AAA22552 | Shibano et al | 1985 | Bt sotto | |
| Cry1Aa3 | BAA00257 | Shimizu et al | 1988 | Bt aizawai IPL7 | |
| Cry1Aa4 | CAA31886 | Masson et al | 1989 | Bt entomocidus | |
| Cry1Aa5 | BAA04468 | Udayasuriyan et al | 1994 | Bt Fu-2-7 | |
| Cry1Aa6 | AAA86265 | Masson et al | 1994 | Bt kurstaki NRD-12 | |
| Cry1Aa7 | AAD46139 | Osman et al | 1999 | Bt C12 | |
| Cry1Aa8 | I26149 | Liu | 1996 | | DNA sequence only |
| Cry1Aa9 | BAA77213 | Nagamatsu et al | 1999 | Bt dendrolimus T84A1 | |
| Cry1Aa10 | AAD55382 | Hou and Chen | 1999 | Bt kurstaki HD-1-02 | |
| Cry1Aa11 | CAA70856 | Tounsi et al | 1999 | Bt kurstaki | |
| Cry1Aa12 | AAP80146 | Yao et al | 2001 | Bt Ly30 | |
| Cry1Aa13 | AAM44305 | Zhong et al | 2002 | Bt sotto | |
| Cry1Aa14 | AAP40639 | Ren et al | 2002 | unpublished | |
| Cry1Aa15 | AAY66993 | Sauka et al | 2005 | Bt INTA Mol-12 | |
| Cry1Ab1 | AAA22330 | Wabiko et al | 1986 | Bt berliner 1715 | |
| Cry1Ab2 | AAA22613 | Thorne et al | 1986 | Bt kurstaki | |
| Cry1Ab3 | AAA22561 | Geiser et al | 1986 | Bt kurstaki HD1 | |
| Cry1Ab4 | BAA00071 | Kondo et al | 1987 | Bt kurstaki HD1 | |
| Cry1Ab5 | CAA28405 | Hofte et al | 1986 | Bt berliner 1715 | |
| Cry1Ab6 | AAA22420 | Hefford et al | 1987 | Bt kurstaki NRD-12 | |
| Cry1Ab7 | CAA31620 | Haider & Ellar | 1988 | Bt aizawai IC1 | |
| Cry1Ab8 | AAA22551 | Oeda et al | 1987 | Bt aizawai IPL7 | |
| Cry1Ab9 | CAA38701 | Chak & Jen | 1993 | Bt aizawai HD133 | |
| Cry1Ab10 | A29125 | Fischhoff et al | 1987 | Bt kurstaki HD1 | |
| Cry1Ab11 | I12419 | Ely & Tippett | 1995 | Bt A20 | DNA sequence only |
| Cry1Ab12 | AAC64003 | Silva-Werneck et al | 1998 | Bt kurstaki S93 | |
| Cry1Ab13 | AAN76494 | Tan et al | 2002 | Bt c005 | |
| Cry1Ab14 | AAG16877 | Meza-Basso & Theoduloz | 2000 | Native Chilean Bt | |
| Cry1Ab15 | AAO13302 | Li et al | 2001 | Bt B-Hm-16 | |
| Cry1Ab16 | AAK55546 | Yu et al | 2002 | Bt AC-11 | |
| Cry1Ab17 | AAT46415 | Huang et al | 2004 | Bt WB9 | |
| Cry1Ab18 | AAQ88259 | Stobdan et al | 2004 | Bt | |
| Cry1Ab19 | AAW31761 | Zhong et al | 2005 | Bt X-2 | |
| Cry1Ab20 | ABB72460 | Liu et al | 2006 | BtC008 | |
| Cry1Ab21 | ABS18384 | Swiecicka et al | 2007 | BtIS5056 | |
| Cry1Ab22 | ABW87320 | Wu and Feng | 2008 | BtS2491Ab | |
| Cry1Ab-like | AAK14336 | Nagarathinam et al | 2001 | Bt kunthala RX24 | uncertain sequence |
| Cry1Ab-like | AAK14337 | Nagarathinam et al | 2001 | Bt kunthala RX28 | uncertain sequence |
| Cry1Ab-like | AAK14338 | Nagarathinam et al | 2001 | Bt kunthala RX27 | uncertain sequence |
| Cry1Ab-like | ABG88858 | Lin et al | 2006 | Bt ly4a3 | insufficient sequence |
| Cry1Ac1 | AAA22331 | Adang et al | 1985 | Bt kurstaki HD73 | |
| Cry1Ac2 | AAA22338 | Von Tersch et al | 1991 | Bt kenyae | |
| Cry1Ac3 | CAA38098 | Dardenne et al | 1990 | Bt BTS89A | |
| Cry1Ac4 | AAA73077 | Feitelson | 1991 | Bt kurstaki PS85A1 | |
| Cry1Ac5 | AAA22339 | Feitelson | 1992 | Bt kurstaki PS81GG | |
| Cry1Ac6 | AAA86266 | Masson et al | 1994 | Bt kurstaki NRD-12 | |
| Cry1Ac7 | AAB46989 | Herrera et al | 1994 | Bt kurstaki HD73 | |
| Cry1Ac8 | AAC44841 | Omolo et al | 1997 | Bt kurstaki HD73 | |
| Cry1Ac9 | AAB49768 | Gleave et al | 1992 | Bt DSIR732 | |
| Cry1Ac10 | CAA05505 | Sun | 1997 | Bt kurstaki YBT-1520 | |
| Cry1Ac11 | CAA10270 | Makhdoom & Riazuddin | 1998 | | |
| Cry1Ac12 | I12418 | Ely & Tippett | 1995 | Bt A20 | DNA sequence only |
| Cry1Ac13 | AAD38701 | Qiao et al | 1999 | Bt kurstaki HD1 | |
| Cry1Ac14 | AAQ06607 | Yao et al | 2002 | Bt Ly30 | |
| Cry1Ac15 | AAN07788 | Tzeng et al | 2001 | Bt from Taiwan | |
| Cry1Ac16 | AAU87037 | Zhao et al | 2005 | Bt H3 | |
| Cry1Ac17 | AAX18704 | Hire et al | 2005 | Bt kenyae HD549 | |
| Cry1Ac18 | AAY88347 | Kaur & Allam | 2005 | Bt SK-729 | |
| Cry1Ac19 | ABD37053 | Gao et al | 2005 | Bt C-3 | |
| Cry1Ac20 | ABB89046 | Tan et al | 2005 | | |
| Cry1Ac21 | AAY66992 | Sauka et al | 2005 | INTA Mol-12 | |
| Cry1Ac22 | ABZ01836 | Zhang & Fang | 2008 | Bt WO5-1 | |
| Cry1Ac23 | CAQ30431 | Kashyap et al | 2008 | Bt | |
| Cry1Ac24 | ABL01535 | Arango et al | 2008 | Bt 146-158-01 | |
| Cry1Ac25 | FJ513324 | Guan Peng et al | 2008 | Bt Tm37-6 | No NCBI link July 09 |
| Cry1Ac26 | FJ617446 | Guan Peng et al | 2009 | Bt Tm41-4 | No NCBI link July 09 |
| Cry1Ac27 | FJ617447 | Guan Peng et al | 2009 | Bt Tm44-1B | No NCBI link July 09 |
| Cry1Ac28 | ACM90319 | Li et al | 2009 | Bt Q-12 | |
| Cry1Ad1 | AAA22340 | Feitelson | 1993 | Bt aizawai PS81I | |
| Cry1Ad2 | CAA01880 | Anonymous | 1995 | Bt PS81RR1 | |
| Cry1Ae1 | AAA22410 | Lee & Aronson | 1991 | Bt alesti | |
| Cry1Af1 | AAB82749 | Kang et al | 1997 | Bt NT0423 | |
| Cry1Ag1 | AAD46137 | Mustafa | 1999 | | |
| Cry1Ah1 | AAQ14326 | Tan et al | 2000 | | |
| Cry1Ah2 | ABB76664 | Qi et al | 2005 | Bt alesti | |
| Cry1Ai1 | AAO39719 | Wang et al | 2002 | | |
| Cry1A-like | AAK14339 | Nagarathinam et al | 2001 | Bt kunthala nags3 | uncertain sequence |
| Cry1Ba1 | CAA29898 | Brizzard & Whiteley | 1988 | Bt thuringiensis HD2 | |
| Cry1Ba2 | CAA65003 | Soetaert | 1996 | Bt entomocidus HD110 | |
| Cry1Ba3 | AAK63251 | Zhang et al | 2001 | | |
| Cry1Ba4 | AAK51084 | Nathan et al | 2001 | Bt entomocidus HD9 | |
| Cry1Ba5 | ABO20894 | Song et al | 2007 | Bt sfw-12 | |
| Cry1Ba6 | ABL60921 | Martins et al | 2006 | Bt S601 | |
| Cry1Bb1 | AAA22344 | Donovan et al | 1994 | Bt EG5847 | |
| Cry1Bc1 | CAA86568 | Bishop et al | 1994 | Bt morrisoni | |
| Cry1Bd1 | AAD10292 | Kuo et al | 2000 | Bt wuhanensis HD525 | |
| Cry1Bd2 | AAM93496 | Isakova et al | 2002 | Bt 834 | |
| Cry1Be1 | AAC32850 | Payne et al | 1998 | Bt PS158C2 | |
| Cry1Be2 | AAQ52387 | Baum et al | 2003 | | |
| Cry1Be3 | FJ716102 | Xiaodong Sun et al | 2009 | Bt | No NCBI link July 09 |
| Cry1Bf1 | CAC50778 | Arnaut et al | 2001 | | |
| Cry1Bf2 | AAQ52380 | Baum et al | 2003 | | |
| Cry1Bg1 | AAO39720 | Wang et al | 2002 | | |
| Cry1Ca1 | CAA30396 | Honee et al | 1988 | Bt entomocidus 60.5 | |
| Cry1Ca2 | CAA31951 | Sanchis et al | 1989 | Bt aizawai 7.29 | |
| Cry1Ca3 | AAA22343 | Feitelson | 1993 | Bt aizawai PS81I | |
| Cry1Ca4 | CAA01886 | Van Mellaert et al | 1990 | Bt entomocidus HD110 | |
| Cry1Ca5 | CAA65457 | Strizhov | 1996 | Bt aizawai 7.29 | |
| Cry1Ca6 | AAF37224 | Yu et al | 2000 | Bt AF-2 | |
| Cry1Ca7 | AAG50438 | Aixing et al | 2000 | Bt J8 | |
| Cry1Ca8 | AAM00264 | Chen et al | 2001 | Bt c002 | |
| Cry1Ca9 | AAL79362 | Kao et al | 2003 | Bt G10-01A | |
| Cry1Ca10 | AAN16462 | Lin et al | 2003 | Bt E05-20a | |
| Cry1Ca11 | AAX53094 | Cai et al | 2005 | Bt C-3 | |
| Cry1Cb1 | M97880 | Kalman et al | 1993 | Bt galleriae HD29 | DNA sequence only |
| Cry1Cb2 | AAG35409 | Song et al | 2000 | Bt c001 | |
| Cry1Cb3 | ACD50894 | Huang et al | 2008 | Bt 087 | |
| Cry1Cb-like | AAX63901 | Thammasittirong et al | 2005 | Bt TA476-1 | insufficient sequence |
| Cry1Da1 | CAA38099 | Hofte et al | 1990 | Bt aizawai HD68 | |
| Cry1Da2 | 176415 | Payne & Sick | 1997 | | DNA sequence only |
| Cry1Db1 | CAA80234 | Lambert | 1993 | Bt BTS00349A | |
| Cry1Db2 | AAK48937 | Li et al | 2001 | Bt B-Pr-88 | |
| Cry1Dc1 | ABK35074 | Lertwiriyawong et al | 2006 | BtJC291 | |
| Cry1Ea1 | CAA37933 | Visser et al | 1990 | Bt kenyae 4F1 | |
| Cry1Ea2 Ea2 | CAA39609 | Bosse et al | 1990 | Bt kenyae | |
| Cry1Ea3 | AAA22345 | Feitelson | 1991 | Bt kenyae PS81F | |
| Cry1Ea4 | AAD04732 | Barboza-Corona et al | 1998 | Bt kenyae LBIT-147 | |
| Cry1Ea5 | A15535 | Botterman et al | 1994 | | DNA sequence only |
| Cry1Ea6 | AAL50330 | Sun et al | 1999 | Bt YBT-032 | |
| Cry1Ea7 | AAW72936 | Huehne et al | 2005 | Bt JC190 | |
| Cry1Ea8 | ABX11258 | Huang et al | 2007 | Bt HZM2 | |
| Cry1Eb1 | AAA22346 | Feitelson | 1993 | Bt aizawai PS81A2 | |
| Cry1Fa1 | AAA22348 | Chambers et al | 1991 | Bt aizawai EG6346 | |
| Cry1Fa2 | AAA22347 | Feitelson | 1993 | Bt aizawai PS81I | |
| Cry1Fb1 | CAA80235 | Lambert | 1993 | Bt BTS00349A | |
| Cry1Fb2 | BAA25298 | Masuda & Asano | 1998 | Bt morrisoni INA67 | |
| Cry1Fb3 | AAF21767 | Song et al | 1998 | Bt morrisoni | |
| Cry1Fb4 | AAC10641 | Payne et al | 1997 | | |
| Cry1Fb5 | AAO13295 | Li et al | 2001 | Bt B-Pr-88 | |
| Cry1Fb6 | ACD50892 | Huang et al | 2008 | Bt 012 | |
| Cry1Fb7 | ACD50893 | Huang et al | 2008 | Bt 087 | |
| Cry1Ga1 | CAA80233 | Lambert | 1993 | Bt BTS0349A | |
| Cry1Ga2 | CAA70506 | Shevelev et al | 1997 | Bt wuhanensis | |
| Cry1Gb1 | AAD10291 | Kuo & Chak | 1999 | Bt wuhanensis | |
| | | | | HD525 | |
| Cry1Gb2 | AAO13756 | Li et al | 2000 | Bt B-Pr-88 | |
| Cry1Gc | AAQ52381 | Baum et al | 2003 | | |
| Cry1Ha1 | CAA80236 | Lambert | 1993 | Bt BTS02069AA | |
| Cry1Hb1 | AAA79694 | Koo et al | 1995 | Bt morrisoni BF190 | |
| Cry1H-like | AAF01213 | Srifah et al | 1999 | Bt JC291 | insufficient sequence |
| Cry1Ia1 | CAA44633 | Tailor et al | 1992 | Bt kurstaki | |
| Cry1Ia2 | AAA22354 | Gleave et al | 1993 | Bt kurstaki | |
| Cry1Ia3 | AAC36999 | Shin et al | 1995 | Bt kurstaki HD1 | |
| Cry1Ia4 | AAB00958 | Kostichka et al | 1996 | Bt AB88 | |
| Cry1Ia5 | CAA70124 | Selvapandiyan | 1996 | Bt 61 | |
| Cry1Ia6 | AAC26910 | Zhong et al | 1998 | Bt kurstaki S101 | |
| Cry1Ia7 | AAM73516 | Porcar et al | 2000 | Bt | |
| Cry1Ia8 | AAK66742 | Song et al | 2001 | | |
| Cry1Ia9 | AAQ08616 | Yao et al | 2002 | Bt Ly30 | |
| Cry1Ia10 | AAP86782 | Espindola et al | 2003 | Bt thuringiensis | |
| Cry1Ia11 | CAC85964 | Tounsi et al | 2003 | Bt kurstaki BNS3 | |
| Cry1Ia12 | AAV53390 | Grossi de Sa et al | 2005 | Bt | |
| Cry1Ia13 | ABF83202 | Martins et al | 2006 | Bt | |
| Cry1Ia14 | ACG63871 | Liu & Guo | 2008 | Bt11 | |
| Cry1Ia15 | FJ617445 | Guan Peng et al | 2009 | Bt E-1B | No NCBI link July 2009 |
| Cry1Ia16 | FJ617448 | Guan Peng et al | 2009 | Bt E-1A | No NCBI link July 2009 |
| Cry1Ib1 | AAA82114 | Shin et al | 1995 | Bt entomocidus BP465 | |
| Cry1Ib2 | ABW88019 | Guan et al | 2007 | BtPP61 | |
| Cry1Ib3 | ACD75515 | Liu & Guo | 2008 | Bt GS8 | |
| Cry1Ic1 | AAC62933 | Osman et al | 1998 | Bt C18 | |
| Cry1Ic2 | AAE71691 | Osman et al | 2001 | | |
| Cry1Id1 | AAD44366 | Choi | 2000 | | |
| Cry1Ie1 | AAG43526 | Song et al | 2000 | Bt BTC007 | |
| Cry1If1 | AAQ52382 | Baum et al | 2003 | | |
| Cry1I-like | AAC31094 | Payne et al | 1998 | | insufficient sequence |
| Cry1I-like | ABG88859 | Lin & Fang | 2006 | Bt ly4a3 | insufficient sequence |
| Cry1Ja1 | AAA22341 | Donovan | 1994 | Bt EG5847 | |
| Cry1Jb1 | AAA98959 | Von Tersch & Gonzalez | 1994 | Bt EG5092 | |
| Cry1Jc1 | AAC31092 | Payne et al | 1998 | | |
| Cry1Jc2 | AAQ52372 | Baum et al | 2003 | | |
| Cry1Jd1 | CAC50779 | Arnaut et al | 2001 | Bt | |
| Cry1Ka1 | AAB00376 | Koo et al | 1995 | Bt morrisoni BF190 | |
| Cry1La1 | AAS60191 | Je et al | 2004 | Bt kurstaki K1 | |
| Cry1-like | AAC31091 | Payne et al | 1998 | | insufficient sequence |
| Cry2Aa1 | AAA22335 | Donovan et al | 1989 | Bt kurstaki | |
| Cry2Aa2 | AAA83516 | Widner & Whiteley | 1989 | Bt kurstaki HD1 | |
| Cry2Aa3 | D86064 | Sasaki et al | 1997 | Bt sotto | DNA sequence only |
| Cry2Aa4 | AAC04867 | Misra et al | 1998 | Bt kenyae HD549 | |
| Cry2Aa5 | CAA10671 | Yu & Pang | 1999 | Bt SL39 | |
| Cry2Aa6 | CAA10672 | Yu & Pang | 1999 | Bt YZ71 | |
| Cry2Aa7 | CAA10670 | Yu & Pang | 1999 | Bt CY29 | |
| Cry2Aa8 | AAO13734 | Wei et al | 2000 | Bt Dongbei 66 | |
| Cry2Aa9 | AAO13750 | Zhang et al | 2000 | | |
| Cry2Aa10 | AAQ04263 | Yao et al | 2001 | | |
| Cry2Aa11 | AAQ52384 | Baum et al | 2003 | | |
| Cry2Aa12 | ABI83671 | Tan et al | 2006 | Bt Rpp39 | |
| Crv2Aa13 | ABL01536 | Arango et al | 2008 | But 146-158-01 | |
| Cry2Aa14 | ACF04939 | Hire et al | 2008 | Bt HD-550 | |
| Cry2Ab1 | AAA22342 | Widner & Whiteley | 1989 | Bt kurstaki HD1 | |
| Cry2Ab2 | CAA39075 | Dankocsik et al | 1990 | Bt kurstaki HD1 | |
| Cry2Ab3 | AAG36762 | Chen et al | 1999 | Bt BTC002 | |
| Cry2Ab4 | AAO13296 | Li et al | 2001 | Bt B-Pr-88 | |
| Cry2Ab5 | AAQ04609 | Yao et al | 2001 | Bt ly30 | |
| Cry2Ab6 | AAP59457 | Wang et al | 2003 | Bt WZ-7 | |
| Cry2Ab7 | AAZ66347 | Udayasuriyan et al | 2005 | Bt 14-1 | |
| Cry2Ab8 | ABC95996 | Huang et al | 2006 | Bt WB2 | |
| Cry2Ab9 | ABC74968 | Zhang et al | 2005 | Bt LLB6 | |
| Cry2Ab10 | EF157306 | Lin et al | 2006 | Bt LyD | |
| Cry2Ab11 | CAM84575 | Saleem et al | 2007 | Bt CMBL-BT1 | |
| Cry2Ab12 | ABM21764 | Lin et al | 2007 | Bt LyD | |
| Cry2Ab13 | ACG76120 | Zhu et al | 2008 | Bt ywc5-4 | |
| Cry2Ab14 | ACG76121 | Zhu et al | 2008 | Bt Bts | |
| Cry2Ac1 | CAA40536 | Aronson | 1991 | Bt shanghai S1 | |
| Cry2Ac2 | AAG35410 | Song et al | 2000 | | |
| Cry2Ac3 | AAQ52385 | Baum et al | 2003 | | |
| Cry2Ac4 | ABC95997 | Huang et al | 2006 | Bt WB9 | |
| Cry2Ac5 | ABC74969 | Zhang et al | 2005 | | |
| Cry2Ac6 | ABC74793 | Xia et al | 2006 | Bt wuhanensis | |
| Cry2Ac7 | CAL18690 | Saleem et al | 2008 | Bt SBSBT-1 | |
| Cry2Ac8 | CAM09325 | Saleem et al | 2007 | Bt CMBL-BT1 | |
| Cry2Ac9 | CAM09326 | Saleem et al | 2007 | Bt CMBL-BT2 | |
| Cry2Ac10 | ABN15104 | Bai et al | 2007 | Bt QCL-1 | |
| Cry2Ac11 | CAM83895 | Saleem et al | 2007 | Bt HD29 | |
| Cry2Ac12 | CAM83896 | Saleem et al | 2007 | Bt CMBL-BT3 | |
| Cry2Ad1 | AAF09583 | Choi et al | 1999 | Bt BR30 | |
| Cry2Ad2 | ABC86927 | Huang et al | 2006 | Bt WB10 | |
| Cry2Ad3 | CAK29504 | Saleem et al | 2006 | Bt 5_2AcT(1) | |
| Cry2Ad4 | CAM32331 | Saleem et al | 2007 | Bt CMBL-BT2 | |
| Cry2Ad5 | CAO78739 | Saleem et al | 2007 | Bt HD29 | |
| Cry2Ae1 | AAQ52362 | Baum et al | 2003 | | |
| Cry2Af1 | ABO30519 | Beard et al | 2007 | Bt C81 | |
| Cry2Ag | ACH91610 | Zhu et al | 2008 | Bt JF19-2 | |
| Cry2Ah | EU939453 | Zhang et al | 2008 | Bt | No NCBI link July 09 |
| Cry2Ah2 | ACL80665 | Zhang et al | 2009 | Bt BRC-ZQL3 | |
| Cry2Ai | FJ788388 | Udayasuriyan et al | 2009 | Bt | No NCBI link July 09 |
| Cry3Aa1 | AAA22336 | Herrnstadt et al | 1987 | Bt san diego | |
| Cry3Aa2 | AAA22541 | Sekar et al | 1987 | Bt tenebrionis | |
| Cry3Aa3 | CAA68482 | Hofte et al | 1987 | | |
| Cry3Aa4 | AAA22542 | McPherson et al | 1988 | Bt tenebrionis | |
| Cry3Aa5 | AAA50255 | Donovan et al | 1988 | Bt morrisoni EG2158 | |
| Cry3Aa6 | AAC43266 | Adams et al | 1994 | Bt tenebrionis | |
| Cry3Aa7 | CAB41411 | Zhang et al | 1999 | Bt 22 | |
| Cry3Aa8 | AAS79487 | Gao and Cai | 2004 | Bt YM-03 | |
| Cry3Aa9 | AAWO659 | Bulla and Candas | 2004 | Bt UTD-001 | |
| Cry3Aa10 | AAU29411 | Chen et al | 2004 | Bt 886 | |
| Cry3Aa11 | AAW82872 | Kurt et al | 2005 | Bt tenebrionis Mm2 | |
| Cry3Aa12 | ABY49136 | Sezen et al | 2008 | Bt tenebrionis | |
| Cry3Ba1 | CAA34983 | Sick et al | 1990 | Bt tolworthi 43F | |
| Cry3Ba2 | CAA00645 | Peferoen et al | 1990 | Bt PGSI208 | |
| Cry3Bb1 | AAA22334 | Donovan et al | 1992 | Bt EG4961 | |
| Cry3Bb2 | AAA74198 | Donovan et al | 1995 | Bt EG5144 | |
| Cry3Bb3 | I15475 | Peferoen et al | 1995 | | DNA sequence only |
| Cry3Ca1 | CAA42469 | Lambert et al | 1992 | Bt kurstaki BtI109P | |
| Cry4Aa1 | CAA68485 | Ward & Ellar | 1987 | Bt israelensis | |
| Cry4Aa2 | BAA00179 | Sen et al | 1988 | Bt israelensis HD522 | |
| Cry4Aa3 | CAD30148 | Berry et al | 2002 | Bt israelensis | |
| Cry4A-like | AAY96321 | Mahalakshmi et al | 2005 | Bt LDC-9 | insufficient sequence |
| Cry4Ba1 | CAA30312 | Chungjatpornchai et al | 1988 | Bt israelensis 4Q2-72 | |
| Cry4Ba2 | CAA30114 | Tungpradubkul et al | 1988 | Bt israelensis | |
| Cry4Ba3 | AAA22337 | Yamamoto et al | 1988 | Bt israelensis | |
| Cry4Ba4 | BAA00178 | Sen et al | 1988 | Bt israelensis HD522 | |
| Cry4Ba5 | CAD30095 | Berry et al | 2002 | Bt israelensis | |
| Cry4Ba-like | ABC47686 | Mahalakshmi et al | 2005 | Bt LDC-9 | insufficient sequence |
| Cry4Ca1 | EU646202 | Shu et al | 2008 | | No NCBI link July 09 |
| Cry4Cb1 | FJ403208 | Jun & Furong | 2008 | Bt HS18-1 | No NCBI link July 09 |
| Cry4Cb2 | FJ597622 | Jun & Furong | 2008 | Bt Ywc2-8 | No NCBI link July 09 |
| Cry4Cc1 | FJ403207 | Jun & Furong | 2008 | Bt MC28 | No NCBI link July 09 |
| Cry5Aa1 | AAA67694 | Narva et al | 1994 | Bt darmstadiensis PS17 | |
| Cry5Ab1 | AAA67693 | Narva et al | 1991 | Bt darmstadiensis PS17 | |
| Cry5Ac1 | I34543 | Payne et al | 1997 | | DNA sequence only |
| Cry5Ad1 | ABQ82087 | Lenane et al | 2007 | Bt L366 | |
| Cry5Ba1 | AAA68598 | Foncerrada & Narva | 1997 | Bt PS86Q3 | |
| Cry5Ba2 | ABW88932 | Guo et al | 2008 | YBT 1518 | |
| Cry6Aa1 | AAA22357 | Narva et al | 1993 | Bt PS52A1 | |
| Cry6Aa2 | AAM46849 | Bai et al | 2001 | YBT 1518 | |
| Cry6Aa3 | ABH03377 | Jia et al | 2006 | Bt 96418 | |
| Cry6Ba1 | AAA22358 | Narva et al | 1991 | Bt PS69D1 | |
| Cry7Aa1 | AAA22351 | Lambert et al | 1992 | Bt galleriae PGSI245 | |
| Cry7Ab1 | AAA21120 | Narva & Fu | 1994 | Bt dakota HD511 | |
| Cry7Ab2 | AAA21121 | Narva & Fu | 1994 | Bt kumamotoensis 867 | |
| Cry7Ab3 | ABX24522 | Song et al | 2008 | Bt WZ-9 | |
| Cry7Ab4 | EU380678 | Shu et al | 2008 | Bt | No NCBI link July 09 |
| Cry7Ab5 | ABX79555 | Aguirre-Arzola et al | 2008 | Bt monterrey GM-33 | |
| Cry7Ab6 | ACI44005 | Deng et al | 2008 | Bt HQ122 | |
| Cry7Ab7 | FJ940776 | Wang et al | 2009 | | No NCBI link Sept 09 |
| Cry7Ab8 | GU145299 | Feng Jing | 2009 | | No NCBI link Nov 09 |
| Cry7Ba1 | ABB70817 | Zhang et al | 2006 | Bt huazhongensis | |
| Cry7Ca1 | ABR67863 | Gao et al | 2007 | Bt BTH-13 | |
| Cry7Da1 | ACQ99547 | Yi et al | 2009 | Bt LH-2 | |
| Cry8Aa1 | AAA21117 | Narva & Fu | 1992 | Bt kumamotoensis | |
| Cry8Ab1 | EU044830 | Cheng et al | 2007 | Bt B-JJX | No NCBI link July 09 |
| Cry8Ba1 | AAA21118 | Narva & Fu | 1993 | Bt kumamotoensis | |
| Cry8Bb1 | CAD57542 | Abad et al | 2002 | | |
| Cry8Bc1 | CAD57543 | Abad et al | 2002 | | |
| Cry8Ca1 | AAA21119 | Sato et al. | 1995 | Bt japonensis Buibui | |
| Cry8Ca2 | AAR98783 | Shu et al | 2004 | Bt HBF-1 | |
| Cry8Ca3 | EU625349 | Du et al | 2008 | Bt FTL-23 | No NCBI link July 09 |
| Cry8Da1 | BAC07226 | Asano et al | 2002 | Bt galleriae | |
| Cry8Da2 | BD133574 | Asano et al | 2002 | Bt | DNA sequence only |
| Cry8Da3 | BD133575 | Asano et al | 2002 | Bt | DNA sequence only |
| Cry8Db1 | BAF93483 | Yamaguchi et al | 2007 | Bt BBT2-5 | |
| Cry8Ea1 | AAQ73470 | Fuping et al | 2003 | Bt 185 | |
| Cry8Ea2 | EU047597 | Liu et al | 2007 | Bt B-DLL | No NCBI link July 09 |
| Cry8Fa1 | AAT48690 | Shu et al | 2004 | Bt 185 | also AAW81032 |
| Cry8Ga1 | AAT46073 | Shu et al | 2004 | Bt HBF-18 | |
| Cry8Ga2 | ABC42043 | Yan et al | 2008 | Bt 145 | |
| Cry8Ga3 | FJ198072 | Xiaodong et al | 2008 | Bt FCD114 | No NCBI link July 09 |
| Cry8Ha1 | EF465532 | Fuping et al | 2006 | Bt 185 | No NCBI link July 09 |
| Cry8Ia1 | EU381044 | Yan et al | 2008 | Bt su4 | No NCBI link July 09 |
| Cry8Ja1 | EU625348 | Du et al | 2008 | Bt FPT-2 | No NCBI link July 09 |
| Cry8Ka1 | FJ422558 | Quezado et al | 2008 | | No NCBI link July 09 |
| Cry8Ka2 | ACN87262 | Noguera & Ibarra | 2009 | Bt kenyae | |
| Cry8-like | FJ770571 | Noguera & Ibarra | 2009 | Bt canadensis | DNA sequence only |
| Cry8-like | ABS53003 | Mangena et al | 2007 | Bt | |
| Cry9Aa1 | CAA41122 | Shevelev et al | 1991 | Bt galleriae | |
| Cry9Aa2 | CAA41425 | Gleave et al | 1992 | Bt DSIR517 | |
| Cry9Aa3 | GQ249293 | Su et al | 2009 | Bt SC5(D2) | No NCBI link July 09 |
| Cry9Aa4 | GQ249294 | Su et al | 2009 | Bt T03C001 | No NCBI link July 09 |
| Cry9Aa like | AAQ52376 | al Baum et | 2003 | | incomplete sequence |
| Cry9Ba1 | CAA52927 | Shevelev et al | 1993 | Bt galleriae | |
| Cry9Bb1 | AAV28716 | Silva-Werneck et al | 2004 | Btjaponensis | |
| Cry9Ca1 | CAA85764 | Lambert et al | 1996 | Bt tolworthi | |
| Cry9Ca2 | AAQ52375 | Baum et al | 2003 | | |
| Cry9Da1 | BAA 19948 | Asano | 1997 | Bt japonensis N141 | |
| Cry9Da2 | AAB97923 | Wasano & Ohba | 1998 | Btjaponensis | |
| Cry9Da3 | GQ249295 | Su et al | 2009 | Bt T03B001 | No NCBI link July 09 |
| Cry9Da4 | GQ249297 | Su et al | 2009 | Bt T03B001 | No NCBI link July 09 |
| Cry9Db1 | AAX78439 | Flannagan & Abad | 2005 | Bt kurstaki DP1019 | |
| Cry9Ea1 | BAA34908 | Midoh & Oyama | 1998 | Bt aizawai SSK-10 | |
| Cry9Ea2 | AAO12908 | Li et al | 2001 | Bt B-Hm-16 | |
| Cry9Ea3 | ABM21765 | Lin et al | 2006 | Bt lyA | |
| Cry9Ea4 | ACE88267 | Zhu et al | 2008 | Bt ywc5-4 | |
| Cry9Ea5 | ACF04743 | Zhu et al | 2008 | Bts | |
| Cry9Ea6 | ACG63872 | Liu & Guo | 2008 | Bt 11 | |
| Cry9Ea7 | FJ380927 | Sun et al | 2008 | | No NCBI link July 09 |
| Cry9Ea8 | GQ249292 | Su et al | 2009 | GQ249292 | No NCBI link July 09 |
| Cry9Eb1 | CAC50780 | Arnaut et al | 2001 | | |
| Cry9Eb2 | GQ249298 | Su et al | 2009 | Bt T03B001 | No NCBI link July 09 |
| Cry9Ec1 | AAC63366 | Wasano et al | 2003 | Bt galleriae | |
| Cry9Ed1 | AAX78440 | Flannagan & Abad | 2005 | Bt kurstaki DP1019 | |
| Cry9Ee1 | GQ249296 | Su et al | 2009 | Bt T03B001 | No NCBI link Aug 09 |
| Cry9-like | AAC63366 | Wasano et al | 1998 | Bt galleriae | insufficient sequence |
| Cry10Aa1 | AAA22614 | Thorne et al | 1986 | Bt israelensis | |
| Cry10Aa2 | E00614 | Aran & Toomasu | 1996 | Bt israelensis ONR-60A | DNA sequence only |
| Cry10Aa3 | CAD30098 | Berry et al | 2002 | Bt israelensis | |
| Cry10Alike | DQ167578 | Mahalakshmi et al | 2006 | Bt LDC-9 | incomplete sequence |
| Cry11Aa1 | AAA22352 | Donovan et al | 1988 | Bt israelensis | |
| Cry11Aa2 | AAA22611 | Adams et al | 1989 | Bt israelensis | |
| Cry11Aa3 | CAD30081 | Berry et al | 2002 | Bt israelensis | |
| Cry11Aa-like | DQ166531 | Mahalakshmi et al | 2007 | Bt LDC-9 | incomplete sequence |
| Cry11Ba1 | CAA60504 | Delecluse et al | 1995 | Bt jegathesan 367 | |
| Cry11Bb1 | AAC97162 | Orduz et al | 1998 | Bt medellin | |
| Cry12Bb1 | AAA22355 | Narva et al | 1991 | Bt PS33F2 | |
| Cry13Bb1 | AAA22356 | Narva et al | 1992 | Bt PS63B | |
| Cry14Aa1 | AAA21516 | Narva et al | 1994 | Bt sotto PS80JJ1 | |
| Cry15Aa1 | AAA22333 | Brown & Whiteley | 1992 | Bt thompsoni | |
| Cry16Aa1 | CAA63860 | Barloy et al | 1996 | Cb malaysia CH18 | |
| Cry17Aa1 | CAA67841 | Barloy et al | 1998 | Cb malaysia CH18 | |
| Cry18Aa1 | CAA67506 | Zhang et al | 1997 | Paenibacillus popilliae | |
| Cry18Ba1 | AAF89667 | Patel et al | 1999 | Paenibacillus popilliae | |
| Cry18Ca1 | AAF89668 | Patel et al | 1999 | Paenibacillus popilliae | |
| Cry19Aa1 | CAA68875 | Rosso & Delecluse | 1996 | Bt jegathesan 367 | |
| Cry19Ba1 | BAA32397 | Hwang et al | 1998 | Bt higo | |
| Cry20Aa1 | AAB93476 | Lee & Gill | 1997 | Bt fukuokaensis | |
| Cry20Ba1 | ACS93601 | Noguera & Ibarra | 2009 | Bt higo LBIT-976 | |
| Cry20-like | GQ144333 | Yi et al | 2009 | Bt Y-5 | DNA sequence only |
| Cry21Aa1 | I32932 | Payne et al | 1996 | | DNA sequence only |
| Cry21Aa2 | I66477 | Feitelson | 1997 | | DNA sequence only |
| Cry21Ba1 | BAC06484 | Sato & Asano | 2002 | Bt roskildiensis | |
| Cry22Aa1 | I34547 | Payne et al | 1997 | | DNA sequence only |
| Cry22Aa2 | CAD43579 | Isaac et al | 2002 | Bt | |
| Cry22Aa3 | ACD93211 | Du et al | 2008 | Bt FZ-4 | |
| Cry22Ab1 | AAK50456 | Baum et al | 2000 | Bt EG4140 | |
| Cry22Ab2 | CAD43577 | Isaac et al | 2002 | Bt | |
| Cry22Ba1 | CAD43578 | Isaac et al | 2002 | Bt | |
| Cry23Aa1 | AAF76375 | Donovan et al | 2000 | Bt | Binary with Cry37Aa1 |
| Cry24Ba1 | AAC61891 | Kawalek and Gill | 1998 | Bt jegathesan | |
| Cry24Ba1 | BAD32657 | Ohgushi et al | 2004 | Bt sotto | |
| Cry24Ca1 | CAJ43600 | Beron & Salerno | 2005 | Bt FCC-41 | |
| Cry25Aa1 | AAC61892 | Kawalek and Gill | 1998 | Bt jegathesan | |
| Cry26Aa1 | AAD25075 | Wojciechowska et al | 1999 | Bt finitimus B-1166 | |
| Cry27Aa1 | BAA82796 | Saitoh | 1999 | Bt higo | |
| Cry28Aa1 | AAD24189 | Wojciechowska et al | 1999 | Bt finitimus B-1161 | |
| | AAG00235 | Moore and Debro | 2000 | Bt finitimus | |
| Cry29Aa1 | CAC80985 | Delecluse et al | 2000 | Bt medellin | |
| Cry30Aa1 | CAC80986 | Delecluse et al | 2000 | Bt medellin | |
| Cry30Ba1 | BAD00052 | Ito et al | 2003 | Bt entomocidus | |
| Cry30Ca1 | BAD67157 | Ohgushi et al | 2004 | Bt sotto | |
| Cry30Ca2 | ACU24781 | Sun and Park | 2009 | Bt jegathesan 367 | |
| Cry30Da1 | EF095955 | Shu et al | 2006 | Bt Y41 | No NCBI link July09 |
| Cry30Db1 | BAE80088 | Kishida et al | 2006 | Bt aizawai BUN1-14 | |
| Cry30Ea1 | ACC95445 | Fang et al | 2007 | BtS2160-1 | |
| Cry30Ea2 | FJ499389 | Jun et al | 2008 | Bt Ywc2-8 | No NCBI link July09 |
| Cry30Fa1 | ACI22625 | Tan et al | 2008 | Bt MC28 | |
| Cry30Ga1 | ACG60020 | Zhu et al | 2008 | Bt HS 18-1 | |
| Cry31Aa1 | BAB11757 | Saitoh & Mizuki | 2000 | Bt 84-HS-1-11 | |
| Cry31Aa2 | AAL87458 | Jung and Cote | 2000 | Bt M15 | |
| Cry31Aa3 | BAE79808 | Uemori et al | 2006 | Bt B0195 | |
| Cry31Aa4 | BAF32571 | Yasutake et al | 2006 | Bt 79-25 | |
| Cry31Aa5 | BAF32572 | Yasutake et al | 2006 | Bt 92-10 | |
| Cry31Ab1 | BAE79809 | Uemori et al | 2006 | Bt B0195 | |
| Cry31Ab2 | BAF32570 | Yasutake et al | 2006 | Bt 31-5 | |
| Cry31Ac1 | BAF34368 | Yasutake et al | 2006 | Bt 87-29 | |
| Cry32Aa1 | AAG36711 | Balasubramanian et al | 2001 | Bt yunnanensis | |
| Cry32Ba1 | BAB78601 | Takebe et al | 2001 | Bt | |
| Cry32Ca1 | BAB78602 | Takebe et al | 2001 | Bt | |
| Cry32Da1 | BAB78603 | Takebe et al | 2001 | Bt | |
| Cry33Aa1 | AAL26871 | Kim et al | 2001 | Bt dakota | |
| Cry34Aa1 | AAG50341 | Ellis et al | 2001 | Bt PS80JJ1 | Binary with Cry35Aa1 |
| Cry34Aa2 | AAK64560 | Rupar et al | 2001 | Bt EG5899 | Binary with Cry35Aa2 |
| Cry34Aa3 | AAT29032 | Schnepf et al | 2004 | Bt PS69Q | Binary with Cry35Aa3 |
| Cry34Aa4 | AAT29030 | Schnepf et al | 2004 | Bt PS185GG | Binary with Cry35Aa4 |
| Cry34Ab1 | AAG41671 | Moellenbeck et al | 2001 | Bt PS149B1 | Binary with Cry35Ab1 |
| Cry34Ac1 | AAG50118 | Ellis et al | 2001 | Bt PS167H2 | Binary with Cry35Ac1 |
| Cry34Ac2 | AAK64562 | Rupar et al | 2001 | Bt EG9444 | Binary with Cry35Ab2 |
| Cry34Ac3 | AAT29029 | Schnepf et al | 2004 | Bt KR1369 | Binary with Cry35Ab3 |
| Cry34Ba1 | AAK64565 | Rupar et al | 2001 | Bt EG4851 | Binary with Cry35Ba1 |
| Cry34Ba2 | AAT29033 | Schnepf et al | 2004 | Bt PS201L3 | Binary with |
| | | | | | Cry35Ba2 |
| Cry34Ba3 | AAT29031 | Schnepf et al | 2004 | Bt PS201HH2 | Binary with Cry35Ba3 |
| Cry35Aa1 | AAG50342 | Ellis et al | 2001 | Bt PS80JJ1 | Binary with Cry34Aa1 |
| Cry35Aa2 | AAK64561 | Rupar et al | 2001 | Bt EG5899 | Binary with Cry34Aa2 |
| Cry35Aa3 | AAT29028 | Schnepf et al | 2004 | Bt PS69Q | Binary with Cry34Aa3 |
| Cry35Aa4 | AAT29025 | Schnepf et al | 2004 | Bt PS185GG | Binary with Cry34Aa4 |
| Cry35Ab1 | AAG41672 | Moellenbeck et al | 2001 | Bt PS149B1 | Binary with Cry34Ab1 |
| Cry35Ab2 | AAK64563 | Rupar et al | 2001 | Bt EG9444 | Binary with Cry34Ac2 |
| Cry35Ab3 | AY536891 | AAT29024 | 2004 | Bt KR1369 | Binary with Cry34Ab3 |
| Cry35Ac1 | AAG50117 | Ellis et al | 2001 | Bt PS167H2 | Binary with Cry34Ac1 |
| Cry35Ba1 | AAK64566 | Rupar et al | 2001 | Bt EG4851 | Binary with Cry34Ba1 |
| Cry35Ba2 | AAT29027 | Schnepf et al | 2004 | BtPS201L3 | Binary with Cry34Ba2 |
| Cry35Ba3 | AAT29026 | Schnepf et al | 2004 | Bt PS201HH2 | Binary with Cry34Ba3 |
| Cry36Aa1 | AAK64558 | Rupar et al | 2001 | Bt | |
| Cry37Aa1 | AAF76376 | Donovan et al | 2000 | Bt | Binary with Cry23Aa |
| Cry38Aa1 | AAK64559 | Rupar et al | 2000 | Bt | |
| Cry39Aa1 | BAB72016 | Ito et al | 2001 | Bt aizawai | |
| Cry40Aa1 | BAB72018 | Ito et al | 2001 | Bt aizawai | |
| Cry40Ba1 | BAC77648 | Ito et al | 2003 | Bun1-14 | |
| Cry40Ca1 | EU381045 | Shu et al | 2008 | Bt Y41 | No NCBI link July09 |
| Cry40Da1 | ACF15199 | Zhang et al | 2008 | Bt S2096-2 | |
| Cry41Aa1 | BAD35157 | Yamashita et al | 2003 | Bt A1462 | |
| Cry41Ab1 | BAD35163 | Yamashita et al | 2003 | Bt A1462 | |
| Cry42Aa1 | BAD35166 | Yamashita et al | 2003 | Bt A1462 | |
| Cry43Aa1 | BAD15301 | Yokoyama and Tanaka | 2003 | P. lentimorbus semadara | |
| Cry43Aa2 | BAD95474 | Nozawa | 2004 | P. popilliae popilliae | |
| Cry43Ba1 | BAD15303 | Yokoyama and Tanaka | 2003 | P. lentimorbus semadara | |
| Cry43-like | BAD15305 | Yokoyama and Tanaka | 2003 | P. lentimorbus semadara | |
| Cry44Aa | BAD08532 | Ito et al | 2004 | Bt entomocidus INA288 | |
| Cry45Aa | BAD22577 | Okumura et al | 2004 | Bt 89-T-34-22 | |
| Cry46Aa | BAC79010 | Ito et al | 2004 | Bt dakota | |
| Cry46Aa2 | BAG68906 | Ishikawa et al | 2008 | Bt A1470 | |
| Cry46Ab | BAD35170 | Yamagiwa et al | 2004 | Bt | |
| Cry47Aa | AAY24695 | Kongsuwan et al | 2005 | Bt CAA890 | |
| Cry48Aa | CAJ18351 | Jones and Berry | 2005 | Bs IAB59 | binary with 49Aa |
| Cry48Aa2 | CAJ86545 | Jones and Berry | 2006 | Bs 47-6B | binary with 49Aa2 |
| Cry48Aa3 | CAJ86546 | Jones and Berry | 2006 | Bs NHA15b | binary with 49Aa3 |
| Crv48Ab | CAJ86548 | Jones and Berry | 2006 | Bs LP1G | binary with 49Ab1 |
| Cry48Ab2 | CAJ86549 | Jones and Berry | 2006 | Bs 2173 | binary with 49Aa4 |
| Cry49Aa | CAH56541 | Jones and Berry | 2005 | Bs IAB59 | binary with 48Aa |
| Cry49Aa2 | CAJ86541 | Jones and Berry | 2006 | Bs 47-6B | binary with 48Aa2 |
| Cry49Aa3 | CAJ86543 | Jones and Berry | 2006 | BsNHA15b | binary with 48Aa3 |
| Cry49Aa4 | CAJ86544 | Jones and Berry | 2006 | Bs 2173 | binary with 48Ab2 |
| Cry49Ab1 | CAJ86542 | Jones and Berry | 2006 | Bs LP1G | binary with 48Ab1 |
| Cry50Aa1 | BAE86999 | Ohgushi et al | 2006 | Bt sotto | |
| Cry51Aa1 | ABI14444 | Meng et al | 2006 | Bt F14-1 | |
| Cry52Aa1 | EF613489 | Song et al | 2007 | Bt Y41 | No NCBI link July09 |
| Cry52Ba1 | FJ361760 | Jun et al | 2008 | Bt BM59-2 | No NCBI link July09 |
| Cry53Aa1 | EF633476 | Song et al | 2007 | Bt Y41 | No NCBI link July09 |
| Cry53Ab1 | FJ361759 | Jun et al | 2008 | Bt MC28 | No NCBI link July09 |
| Cry54Aa1 | ACA52194 | Tan et al | 2009 | Bt MC28 | |
| Cry55Aa1 | ABW88931 | Guo et al | 2008 | YBT 1518 | |
| Cry55Aa2 | AAE33526 | Bradfisch et al | 2000 | BT Y41 | |
| Cry56Aa1 | FJ597621 | Jun & Furong | 2008 | Bt Ywc2-8 | No NCBI link July09 |
| Cry56Aa2 | GQ483512 | Guan Peng et al | 2009 | Bt G7-1 | No NCBI link Aug09 |
| Cry57Aa1 | ANC87261 | Noguera & Ibarra | 2009 | Bt kim | |
| Cry58Aa1 | ANC87260 | Noguera & Ibarra | 2009 | Bt entomocidus | |
| Cry59Aa1 | ACR43758 | Noguera & Ibarra | 2009 | Bt kim LBIT-980 | |

| | | | | | | | |
|---|---|---|---|---|---|---|---|
| **Vip3Aa1** | Vip3Aa | AAC37036 | Estruch et al | 1996 | PNAS 93, 5389-5394 | AB88 | |
| Vip3Aa2 | Vip3Ab | AAC37037 | Estruch et al | 1996 | PNAS 93, 5389-5394 | AB424 | |
| Vip3Aa3 | Vip3Ac | | Estruch et al | 2000 | US 6137033 Oct 2000 | | |
| Vip3Aa4 | PS36A Sup | AAR81079 | Feitelson et al | 1998 | US 6656908 Dec 2003 | Bt PS36A | WO9818 932(A2, A3) 7 May 1998 |
| Vip3Aa5 | PS81F Sup | AAR81080 | Feitelson et al | 1998 | US 6656908 Dec 2003 | Bt PS81F | WO9818 932(A2, A3) 7 May 1998 |
| Vip3Aa6 | Jav90 Sup | AAR81081 | Feitelson et al | 1998 | US 6656908 Dec 2003 | Bt | WO9818 932(A2, A3) 7 May 1998 |
| Vip3Aa7 | Vip83 | AAK95326 | Cai et al | 2001 | unpublished | Bt YBT-833 | |
| Vip3Aa8 | Vip3A | AAK97481 | Loguercio et al | 2001 | unpublished | Bt HD125 | |
| Vip3Aa9 | VipS | CAA76665 | Selvapandiyan et al | 2001 | unpublished | Bt A13 | |
| Vip3Aa10 | Vip3V | AAN60738 | Doss et al | 2002 | Protein Expr. Purif. 26, 82-88 | Bt | |
| Vip3Aa11 | Vip3A | AAR36859 | Liu et al | 2003 | unpublished | Bt C9 | |
| Vip3Aa12 | Vip3A-WB5 | AAM22456 | Wu and Guan | 2003 | unpublished | Bt | |
| Vip3Aa13 | Vip3A | AAL69542 | Chen et al | 2002 | Sheng Wu Gong Cheng Xue Bao 18, 687-692 | Bt S184 | |
| Vip3Aa14 | Vip | AAQ12340 | Polumetla et al | 2003 | unpublished | Bt tolworthi | |
| Vip3Aa15 | Vip3A | AAP51131 | Wu et al | 2004 | unpublished | Bt WB50 | |
| Vip3Aa16 | Vip3LB | AAW65132 | Mesrati et al | 2005 | FEMS Micro Lett 244, 353-358 | Bt | |
| Vip3Aa17 | Jav90 | | Feitelson et al | 1999 | US 6603063 Aug 2003 | Javelin 1990 | WO9957 282(A2, A3) 11Nov 1999 |
| Vip3Aa18 | | AAX49395 | Cai and Xiao | 2005 | unpublished | Bt 9816C | |
| Vip3Aa19 | Vip3ALD | DQ241674 | Liu et al | 2006 | unpublished | Bt AL | |
| Vip3Aa19 | Vip3A-1 | DQ539887 | Hart et al | 2006 | unpublished | | |
| Vip3Aa20 | Vip3A-2 | DQ539888 | Hart et al | 2006 | unpublished | | |
| Vip3Aa21 | Vip | ABD84410 | Panbangred | 2006 | unpublished | Bt aizawai | |
| Vip3Aa22 | Vip3A-LS1 | AAY41427 | Lu et al | 2005 | unpublished | Bt LS1 | |
| Vip3Aa23 | Vip3A-LS8 | AAY41428 | Lu et al | 2005 | unpublished | Bt LS8 | |
| Vip3Aa24 | | BI 880913 | Song et al | 2007 | unpublished | Bt WZ-7 | |
| Vip3Aa25 | | EF608501 | Hsieh et al | 2007 | unpublished | | |
| Vip3Aa26 | | EU294496 | Shen and Guo | 2007 | unpublished | Bt TF9 | |
| Vip3Aa27 | | EU332167 | Shen and Guo | 2007 | unpublished | Bt 16 | |
| Vip3Aa28 | | FJ494817 | Xiumei Yu | 2008 | unpublished | Bt JF23-8 | |
| Vip3Aa29 | | FJ626674 | Xieumei et al | 2009 | unpublished | Bt JF21-1 | |
| Vip3Aa30 | | FJ626675 | Xieumei et al | 2009 | unpublished | MD2-1 | |
| Vip3Aa31 | | FJ626676 | Xieumei et al | 2009 | unpublished | JF21-1 | |
| Vip3Aa32 | | FJ626677 | Xieumei et al | 2009 | unpublished | MD2-1 | |
| . | | | | . | | | |
| **Vip3Ab1** | Vip3B | AAR40284 | Feitelson et al | 1999 | US 6603063 Aug 2003 | Bt KB59A4-6 | WO9957 282(A2, A3) 11Nov 1999 |
| Vip3Ab2 | Vip3D | AAY88247 | Feng and Shen | 2006 | unpublished | Bt | |
| . | | | | . | | | |
| **Vip3Ac1** | PS49C | | Narva et al | . | US application 2004012871 6 | | |
| . | | | | . | | | |
| **Vip3Ad1** | PS158C2 | | Narva et al | | US application 2004012871 6 | | |
| Vip3Ad2 | ISP3B | CAI43276 | Van Rie et al | 2005 | unpublished | Bt | |
| . | | | | . | | | |
| **Vip3Ae1** | ISP3C | CAI43277 | Van Rie et al | 2005 | unpublished | Bt | |
| . | | | | . | | | |
| **Vip3Af1** | ISP3A | CAI43275 | Van Rie et al | 2005 | unpublished | Bt | |
| Vip3Af2 | Vip3C | ADN08753 | Syngenta | . | WO 03/075655 | | |
| . | | | | . | | | |
| **Vip3Ag1** | Vip3B | ADN08758 | Syngenta | . | WO 02/078437 | | |
| Vip3Ag2 | | FJ556803 | Audtho et al | 2008 | | Bt | |
| . | | | | . | | | |
| **Vip3Ah1** | Vip3S | DQ832323 | Li and Shen | 2006 | unpublished | Bt | |
| | | | | | | | |
| **Vip3Ba1** | | AAV70653 | Rang et al | 2004 | unpublished | | |
| **Vip3Bb1** | Vip3Z | ADN08760 | Syngenta | . | WO 03/075655 | | |
| Vip3Bb2 | | EF439819 | Akhurst et al | 2007 | | | |

## Claims

1. A transgenic plant comprising DNA encoding a Vip3Ab insecticidal protein and DNA encoding a Cry1Ca insecticidal protein, said plant further comprising DNA encoding a third insecticidal protein, said third protein being selected from the group consisting of Cry1Fa and Cry1Da.

2. The transgenic plant of claim 1, wherein said third protein is Cry1Fa, said plant further comprising DNA encoding fourth and fifth insecticidal proteins selected from the group consisting of Cry2A, Cry1I, DIG-3, and Cry1Ab.

3. Seed of a plant according to any of claims 1-2, wherein said seed comprises said DNA.

4. A mixture of seeds comprising refuge seeds from non-Bt refuge plants, and a plurality of seeds of claim 3, wherein said refuge seeds comprise between 5% to 40% of all the seeds in the mixture.

5. A plant of any of claims 1-2, wherein said plant is selected from the group consisting of corn, soybeans, and cotton.

6. The plant of claim 5, wherein said plant is a maize plant.

7. The transgenic plant of claim 1, said plant further comprising DNA encoding a Cry1Fa insecticidal protein.

8. A plant cell of a plant of any of claims 1-2, wherein said plant cell comprises said DNA encoding said Vip3Ab insecticidal protein and said DNA encoding said Cry1Ca insecticidal protein, wherein said Vip3Ab insecticidal protein is at least 99% identical with SEQ ID NO: 1, and said Cry1Ca insecticidal protein is at least 99% identical with SEQ ID NO:2.

9. A plant of any of claims 1-2, wherein said Vip3Ab insecticidal protein comprises SEQ ID NO: 1, and said Cry1Ca insecticidal protein comprises SEQ ID NO:2.

10. A method of controlling a fall armyworm insect by contacting said insect with a Vip3Ab insecticidal protein, a Cry1Ca insecticidal protein and an insecticidal protein selected from the group consisting of Cry1Fa and Cry1Da.

## Patentansprüche

1. Eine transgene Pflanze umfassend DNA, welche ein Vip3Ab insektizides Protein codiert und DNA, welche ein Cry1Ca insektizides Protein codiert, wobei besagte Pflanze zusätzlich DNA umfasst, welche ein drittes insektizides Protein codiert, wobei besagtes drittes Protein ausgewählt ist aus der Gruppe bestehend aus Cry1Fa und Cry1Da.

2. Transgene Pflanze gemäß Anspruch 1, wobei besagtes drittes Protein Cry1Fa ist, besagte Pflanze zusätzlich DNA umfasst, welche ein viertes und fünftes Protein codiert, welche ausgewählt sind aus der Gruppe bestehend aus Cry2A, Cry1I, DIG-3, und Cry1Ab.

3. Samen einer Pflanze gemäß einem der Ansprüche 1-2, wobei besagter Samen besagte DNA umfasst.

4. Eine Mischung von Samen umfassend Refuge-Samen von nicht-Bt Refuge-Pflanzen, und eine Vielzahl an Samen gemäß Anspruch 3, wobei besagte Refuge-Samen zwischen 5% bis 40% bezogen auf alle Samen in der Mischung umfasst sind.

5. Pflanze gemäß einem der Ansprüche 1-2, wobei besagte Pflanze ausgewählt ist aus der Gruppe bestehend aus Mais, Sojabohnen und Baumwolle.

6. Pflanze gemäß Anspruch 5, wobei besagte Pflanze eine Maispflanze ist.

7. Transgene Pflanze gemäß Anspruch 1, wobei besagte Pflanze zusätzlich DNA umfasst, welche ein Cry1Fa insektizides Protein codiert.

8. Pflanzenzelle von einer Pflanze gemäß einem der Ansprüche 1-2, wobei besagte Pflanzenzelle besagte DNA, welche ein Vip3Ab insektizides Protein codiert und besagte DNA, welche ein Cry1Ca insektizides Protein codiert umfasst, wobei besagtes Vip3Ab insektizides Protein mindestens 99% identisch zu SEQ ID NO:1, und besagtes Cry1Ca insektizides Protein mindestens 99% identisch zu SEQ ID NO:2 ist.

9. Pflanze gemäß einem der Ansprüche 1-2, wobei besagtes Vip3Ab insektizides Protein SEQ ID NO: 1 umfasst und besagtes Cry1Ca insektizides Protein SEQ ID NO:2 umfasst.

10. Ein Verfahren zur Kontrolle von Heerwurm-Insekten durch Kontaktieren besagter Insekten mit einem Vip3Ab insektizidem Protein, einem Cry1Ca insektizidem Protein und einem insektizidem Protein ausgewählt aus der Gruppe enthaltend Cry1Fa und Cry 1 Da.

## Revendications

1. Végétal transgénique comprenant un ADN codant une protéine insecticide Vip3Ab et un ADN codant une protéine insecticide Cry1Ca, lequel végétal comprend en outre un ADN codant une troisième protéine insecticide, laquelle troisième protéine est choisie dans l'ensemble formé par Cry1Fa et Cry1Da.

2. Végétal transgénique conforme à la revendication 1, dans lequel ladite troisième protéine est Cry1Fa, et lequel végétal comprend en outre un ADN codant une quatrième et une cinquième protéines insecticides choisies dans l'ensemble formé par Cry2A Cry1I, DIG-3 et Cry1Ab.

3. Semence d'un végétal conforme à l'une des revendications 1 et 2, laquelle semence comprend lesdits ADN.

4. Mélange de semences comprenant des semences de refuge issues de plants de refuge non-Bt et des semences conforme à la revendication 3, dans lequel lesdites semences de refuge représentent entre 5 % et 40 % de toutes les semences présentes dans le mélange.

5. Végétal conforme à l'une des revendications 1 et 2, lequel végétal est choisi dans l'ensemble formé par les maïs, soja et coton.

6. Végétal conforme à la revendication 5, lequel végétal est un maïs.

7. Végétal transgénique conforme à la revendication 1, lequel végétal comprend en outre un ADN codant une protéine insecticide Cry1Fa.

8. Cellule végétale d'un végétal conforme à l'une des revendications 1 et 2, laquelle cellule végétale contient ledit ADN codant ladite protéine insecticide Vip3Ab et ledit ADN codant ladite protéine insecticide Cry1Ca, et dans laquelle ladite protéine insecticide Vip3Ab présente une séquence identique, à au moins 99 %, à la Séquence N° 1 et ladite protéine insecticide Cry1Ca présente une séquence identique, à au moins 99 %, à la Séquence N° 2.

9. Végétal conforme à l'une des revendications let 2, dans lequel ladite protéine insecticide Vip3Ab comprend la Séquence N° 1 et ladite protéine insecticide Cry1Ca comprend la Séquence N° 2.

10. Procédé de lutte contre un insecte de type légionnaire d'automne, par mise dudit insecte en contact avec une protéine insecticide Vip3Ab, une protéine insecticide Cry1Ca, et une protéine insecticide choisie dans l'ensemble formé par Cry1Fa et Cry1Da.
